# EUROPÄISCHE PATENTSCHRIFT

(11) **EP 2 724 731 B1**
(45) Veröffentlichungstag und Bekanntmachung des Hinweises auf die Patenterteilung: **23.11.2016**
(21) Anmeldenummer: 13189412.3
(22) Anmeldetag: 18.10.2013
(51) Int. Cl.: A61L 2/08, B65B 55/02, H01J 33/04, B65B 55/08

(54) **Vorrichtung zur Außensterilisation von Kunststoffbehältnissen**
Device for the sterilization of the outside of plastic containers
Dispositif de stérilisation extérieure des conteneurs plastiques

(30) Priorität: 23.10.2012 DE 102012110108
(43) Veröffentlichungstag der Anmeldung: 30.04.2014
(73) Patentinhaber: Krones AG, 93073 Neutraubling (DE)
(72) Erfinder: Knott, Josef, 93073 Neutraubling (DE); Scheuren, Hans, 93073 Neutraubling (DE); Schillinger, Ludwig-Lorenz, 93073 Neutraubling (DE)
(74) Vertreter: Bittner, Bernhard

(56) Entgegenhaltungen:
- EP-A1- 2 149 500
- EP-A1- 2 213 578
- EP-A1- 2 371 397
- EP-A1- 2 594 495
- EP-A2- 2 594 493
- WO-A1-2009/052800

## Beschreibung

Die vorliegende Erfindung bezieht sich auf eine Vorrichtung und ein Verfahren zum Sterilisieren von Kunststoffbehältnissen und insbesondere Kunststoffvorformlingen. Aus dem Stand der Technik ist es bekannt, dass im Rahmen der Herstellung von Getränkebehältnissen teilweise auch die zu befüllenden Behältnisse sterilisiert werden. Dabei wurden im Stand der Technik üblicherweise Sterilisationsverfahren verwendet, welche chemische Mittel, wie etwas Wasserstoffperoxid oder Peressigsäure einsetzen. In jüngerer Zeit ist man jedoch dazu bestrebt, auf den Einsatz derartiger Chemikalien zu verzichten. Entsprechend sind aus dem Stand der Technik auch Vorrichtungen und Verfahren bekannt geworden, bei denen die Sterilisation durch den Einsatz von Strahlungsquellen, wie beispielsweise ultraviolettem Licht oder auch Elektronenstrahlungsquellen, erfolgt. Die vorliegende Erfindung wird daher insbesondere unter Bezugnahme auf Sterilisationseinrichtungen beschrieben, welche die Behältnisse mit Ladungsträgern beaufschlagen, um diese zu sterilisieren.

EP2149500 offenbart einen Elektronenstrahl-Sterilisator für Vorformlinge, welcher eine Dreheinrichtung zur Drehung der Vorformlinge während der Bestrahlung um deren eigene Längsachse aufweist.

EP2371397 lehrt einen Elektronenstrahl-Sterilisator für Vorformlinge, welcher eine Hubeinrichtung für die Vorformlinge aufweist, so dass diese über Strahlfinger geschoben werden, um deren Innensterilisation durchzuführen.

Die aktuellen Systeme, die am Markt erhältlich sind und zur Sterilisation eingesetzt werden, weisen üblicherweise eine Elektronenerzeugungseinrichtung auf, sowie auch einen Strahlfinger, der zur Entkeimung der inneren Oberflächen der Behältnisse dient. Daneben weisen derartige Systeme auch Elektronenerzeugungsvorrichtungen auf, die von außen durch den Behälter auch die inneren Oberflächen behandeln. Bei der Behandlung von außen müssen die Elektronen meist wesentlich stärker beschleunigt werden, da die Wand des zu behandelnden Behältnisses durchdrungen werden muss und trotz der Schwächung müssen die Elektronen immer noch genügend Energie enthalten, um an der inneren Oberfläche sterilisierend zu wirken. Die dabei entstehende Röntgenstrahlung muss durch geeignete Abschirmungen von der Umwelt abgeschirmt werden. Zusätzlich wird das gesamte Behältermaterial mit einer Strahlendosis belastet.

Anordnungen, die mit einem Strahlfinger arbeiten, kommen in der Regel mit wesentlich geringeren Beschleunigungsspannungen und damit mit geringeren Abschirmungen und geringeren Strahlendosen im Behältermaterial aus. Um die Behältnisse auch außen zu sterilisieren, ist allerdings ein weiterer Prozessschritt notwendig. Die Außenbehandlung kann vor, während oder auch nach der Innenbehandlung durchgeführt werden.

Der vorliegenden Erfindung liegt daher die Aufgabe zugrunde, eine Vorrichtung und ein Verfahren zur Verfügung zu stellen, welche eine verbesserte Außensterilisation der Behältnisse und bevorzugt auch eine verbesserte Gesamtsterilisation derselben ermöglichen. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Kunststoffbehältnissen weist eine Transporteinrichtung auf, mittels der die Behältnisse während ihrer Sterilisation entlang eines vorgegebenen Transportpfades transportierbar sind. Weiterhin weist die Vorrichtung einen Reinraum auf, innerhalb dessen die Behältnisse während ihrer Sterilisierung transportierbar sind, wobei dieser Reinraum mittels wenigstens einer Wandung gegenüber einer Umgebung abgegrenzt ist.

Daneben weist die Vorrichtung eine erste Außenbeaufschlagungseinrichtung zum Sterilisieren zumindest eines Abschnittes der Außenwandung der Behältnisse auf, wobei diese Außenbeaufschlagungseinrichtung eine Ladungsträgerquelle zum Erzeugen von Ladungsträgern aufweist, sowie eine erste Bewegungseinrichtung, welche die Behältnisse wenigstens zeitweise während ihrer Sterilisation gegenüber der Außenbeaufschlagungseinrichtung in der Längsrichtung der Behältnisse bewegt.

Erfindungsgemäß weist die Vorrichtung eine Dreheinrichtung auf, weiche die Behältnisse wenigstens zeitweise während deren Sterilisation um deren Längsrichtung dreht, wobei die Dreheinrichtung und die Hubeinrichtung bevorzugt derart ausgestaltet sind, dass eine Drehung der Behältnisse während einer Bewegung der Behältnisse in ihrer Längsrichtung ermöglicht ist oder dass diese Drehungen zeitlich hintereinander ermöglicht sind.

Es wird daher vorgeschlagen, dass die Kunststoffbehältnisse sowohl in ihrer Längsrichtung bewegt werden als auch während dieser Bewegung um ihre eigene Längsachse gedreht werden. Insbesondere können die Behältnisse beim Einfahren bzw. vor oder nach dem Einfahren und/oder beim bzw. vor oder nach dem Ausfahren aus einem Sterilisationsbereich um ihre eigene Achse gedreht werden. Vorteilhaft sind Halteeinrichtungen zum Halten der Behältnisse vorgesehen und besonders bevorzugt greifen oder halten diese Halteeinrichtungen die Behältnisse so, dass weiterhin eine ungehinderte Bestrahlung der Kunststoffbehältnisse möglich ist.

Bei einer weiteren vorteilhaften Ausführungsform weist die Transporteinrichtung einen drehbaren Träger auf. Vorteilhaft ist an diesem drehbaren Träger eine Vielzahl von Halteeinrichtungen zum Halten der Kunststoffbehältnisse angeordnet.

Bei einer weiteren vorteilhaften Ausführungsform weist die Vorrichtung eine mechanische Kopplungseinrichtung auf, welche die Bewegung der Behältnisse in deren Längsrichtung und die Drehbewegung der Behältnisse um deren Längsrichtung miteinander koppelt.

Vorteilhaft weist dabei diese Kopplungseinrichtung wenigstens ein von einer Antriebseinrichtung angetriebenes bzw. antreibbares Kopplungselement auf, welches besonders bevorzugt ein weiteres Kopplungselement antreibt. So könnten beispielsweise als Kopplungselemente zwei ineinandergreifende Verzahnungen etwa von Zahnriemen oder Zahnrädern vorgesehen sein. Eine dieser beiden Verzahnungen könnte dabei von einem Antrieb, wie etwa einem Elektromotor angetrieben sein.

Bei einer weiteren vorteilhaften Ausführungsform ist auch eine weitere Sterilisationseinrichtung vorgesehen, welche die Kunststoffbehältnisse von innen sterilisiert. Diese weitere Sterilisationseinrichtung weist dabei bevorzugt einen stangenartigen Körper auf, der in das Innere der Kunststoffbehältnisse eingeführt wird. Vorteilhaft handelt es sich bei den Kunststoffbehältnissen um sogenannte Kunststoffvorformlinge, die insbesondere durch einen Streckblasvorgang in Kunststoffbehältnisse umgeformt werden.

Vorteilhaft sind der Sterilisationsvorgang für die Außenoberfläche und der Sterilisationsvorgang für die Innenoberfläche zeitlich und/oder örtlich voneinander getrennt. Dies bedeutet, dass eine Sterilisationseinrichtung zum Sterilisieren der Innenoberfläche entlang des Transportpfades der Kunststoffvorformlinge vor oder nach der Sterilisationseinrichtung für die Außenoberflächen angeordnet ist. Der Vorteil bei dieser Trennung der Außenentkeimung von dem Prozess der Innenentkeimung besteht darin, dass eine Behandlung sämtlicher Behältnisoberflächen ohne Einfluss von jeglicher Beschattung durch Behältertransportelemente ermöglicht wird. Vorteilhaft sind daher die Behältnistransportelemente, welche die Behältnisse im Bereich der Außenentkeimung transportieren, mit Greifsystemen ausgestattet, welche die Behälter von Innen greifen.

Bevorzugt werden die Behältnisse wenigstens zeitweise mit einem Innengreifer gehaltert und bevorzugt auch wenigstens zeitweise mit einem Außengreifer. Während die Kunststoffvorformlinge mit Elektronen beaufschlagt werden, sollte sichergestellt werden, dass die Greifelemente keine Schatteneffekte erzeugen, also Oberflächen des Kunststoffvorformlings bedecken, die somit nicht mittels Elektronen sterilisiert werden. Somit könnte beispielsweise während der Außensterilisation ein innengreifer verwendet werden, der keine Außenoberflächen der Behältnisse bedeckt bzw. verschattet.

Vorzugsweise weist eine erfindungsgemäße Vorrichtung zumindest einen Einlaufstern, eine nachfolgende Außensterilisationseinheit, einen Transportstern, der insbesondere als Teilungsverzugsstern ausgebildet ist, um die Kunststoffbehältnisse ausgehend von der Außenentkeimungseinrichtung weiter zu transportieren, eine Innenentkeimungseinrichtung und einen Auslaufstern auf. Dabei sind die genannten Einrichtungen bevorzugt in der hier angegebenen Reihenfolge angeordnet, insbesondere also in der Abfolge Außenentkeimungseinrichtung (bzw. Außensterilisationseinrichtung) - Teilungsverzugsstern - Innennentkeimungseinrichtung (bzw. Innensterilisationseinrichtung). Es wären jedoch auch andere Reihenfolgen denkbar, etwa zunächst eine Innennentkeimungseinrichtung und anschließend eine Außenentkeimungseinrichtung. Daneben wäre es auch möglich, für die Außenentkeimung bzw. Außensterilisation zwei oder mehrere Module bzw. Einrichtungen vorzusehen, welche beispielsweise auf beiden Seiten bezüglich des Transportpfads der Kunststoffvorformlinge angeordnet sein können.

Während des Transports auf dem Einlaufstern werden die Kunststoffvorformlinge bevorzugt von außen gehaltert, beispielsweise unterhalb ihres Tragrings. Anschließend werden die Kunststoffvorformlinge bevorzugt auf Innengreifer übergeben und so an der Mündung bzw. über ihre innenwandung gehalten. Während des Transports durch die Außensterilisation kann somit die gesamte Außenfläche der Kunststoffvorformlinge mit Elektronen beaufschlagt werden. Bei der Übergabe auf den aseptischen Teilungsverzugsstern werden die Kunststoffvorformlinge bevorzugt vom Innengreifer auf einen weiteren Außengreifer übergeben und weiter zur Innenbehandlung transportiert, wo die Kunststoffvorformlinge mittels Außengreifem gehaltert werden.

Hier kann demnach eine komplette Innenentkeimung ohne Beeinträchtigung stattfinden. Der Auslaufstern weist bevorzugt ebenfalls Außengreifer, insb. sog. Doppelklammern auf, damit die Kunststoffvorformlinge von der Innenentkeimung (während derer die Kunststoffvorformlinge bevorzugt mit einer Einfachklammer gehalten werden) optimal und sicher an den Auslauf (hier erfolgt bevorzugt ein Halt der Kunststoffvorformlinge mittels Doppelklammern) übergeben werden können.

Die Kunststoffvorformlinge werden also bevorzugt auf dem gesamten Weg durch das Modul so gegriffen, dass keine Beeinträchtigung der Sterilisation der Oberflächen durch z.B. Klammerelemente auftreten kann.

Vorteilhaft sind für die Sterilisation der Innenoberflächen Greifsysteme vorgesehen, welche die Behältnisse von außen greifen, beispielsweise im Bereich ihrer Mündung oder unterhalb ihres Tragringes. Bevorzugt findet daher die Außensterilisation der Behältnisse vor oder nach der Innensterilisation statt. Besonders bevorzugt findet zuerst die Außensterilisation und anschließend die Innensterilisation statt.

Vorteilhaft sind dabei die verwendeten Greifsysteme, also insbesondere die innenseitigen Greifer und/oder Außengreifer nach hygienischen Gesichtspunkten aufgebaut und sind besonders bevorzugt auch selbst leicht zu sterilisieren. Bei einer vorteilhaften Ausführungsform handelt es sich bei dem Innengreifer um eine Spannzange, die bevorzugt ohne weitere Klemmvorrichtungen die Behältnisse sicher transportieren kann.

Bei einer weiteren vorteilhaften Ausführungsform ist das verwendete Greifsystem zum Greifen der Behältnisse derart ausgestaltet, dass es die Behältnisse an den Übergabestellen innen greifen kann. Bevorzugt ist daher das Greifsystem in der Lage, einen Hub in Richtung der Behälterachse bzw. der oben erwähnten Längsrichtung auszuführen. Dieser Hub kann dabei vorteilhaft mittels eines Kurvensegmentes einer Umlaufkurve, einer pneumatischen Betätigung, einer servoangetriebenen Linearführung oder mit anderen denkbaren Ausführungen ausgeführt werden.

Auch der oben beschriebene Drehantrieb kann, wie oben erwähnt, derart ausgeführt sein, dass jeweils Servomotoren vorgesehen sind. Daneben kann jedoch auch ein zentrales Kurvenzahnrad bzw. ein Zahnriehmen verwendet werden. Es ist auch denkbar das Kurvenzahnrad bzw. den Zahnriemen anzutreiben um beim Abwälzen des Zahnrades am Außenzahnkranz eine Möglichkeit zum Beeinflussen der Drehung zu erhalten (so ist ein Einstellen der gleichmäßigen Doseisverteilung am Umfang des Behältnisses).

Bei einer weiteren vorteilhaften Ausführungsform weist die Kopplungseinrichtung einen Zahnriemen und insbesondere einen flexiblen Zahnriemen auf. Dieser Zahnriemen kann sich dabei entlang einer umlaufenden Linie erstrecken. Bei einer weiteren vorteilhaften Ausführungsform weist die Kopplungseinrichtung wenigstens ein Kopplungselement auf, welches in zwei zueinander entgegengesetzte Bewegungsrichtungen (beispielsweise in zwei unterschiedliche Drehrichtungen oder Umlaufrichtungen) bewegt werden kann. Falls als Kopplungselement ein Zahnriemen vorgesehen ist, kann dieser eine Außenverzahnung oder auch eine Innenverzahnung aufweisen.

Falls das Kopplungselement beispielsweise als Zahnrad ausgeführt ist, kann das Zahnrad (in Umfangsrichtung beidseitig gedreht werden kann. Falls das Kopplungselement als Zahnriemen ausgeführt ist, ist es möglich, dass der Zahnriemen in beide Richtungen bewegt bzw. angetrieben werden kann. Auch ist es dabei möglich, dass das jeweilige angetriebene Kopplungselement mit unterschiedlichen Geschwindigkeiten angetrieben wird.
Durch diese Drehung der Behältnisse während der Außenentkeimung kann eine gleichmäßige Dosis auf die Außenoberfläche der Kunststoffbehältnisse aufgebracht werden. Daneben kann auf diese Weise auch an Sterilisationseinrichtungen selbst gespart werden. Es wäre grundsätzlich möglich, Sterilisationseinrichtungen auf beiden Seiten des Transportpfades der Behältnisse anzubringen. Durch die hier vorgesehene Drehung ist es jedoch möglich, gegebenenfalls auch nur mit einer Sterilisationseinrichtung auszukommen. Auf diese Weise lassen sich einerseits Herstellungskosten sparen, andererseits kann damit auch die TCO (Total Costs of Ownership - Gesamtbetriebskosten) verbessert werden.

Bei einer weiteren vorteilhaften Ausführungsform ist wenigstens ein Element der Dreheinrichtung außerhalb des Reinraumes angeordnet. Bei einer weiteren vorteilhaften Ausführungsform ist auch wenigstens ein Element der Bewegungseinrichtung außerhalb des Reinraumes angeordnet. Dies bedeutet, dass etwaige Antriebsmittel, wie Motoren oder auch Führungskurven bevorzugt außerhalb des Reinraumes angeordnet sind und jeweils die Bewegung in das Innere des Reinraumes übertragen wird. Vorteilhaft ist daher insbesondere nur das Greifsystem mit der eigentlichen Halterung für die Behältnisse in dem Reinraum untergebracht und die Ansteuerungen sind bevorzugt vollständig außerhalb des Reinraumes angeordnet. Das bedeutet, dass das Greifsystem bzw. die gesamte Bewegungseinrichtung durch eine Reinraumgrenze hindurch angeordnet sein kann.

Bei einer weiteren vorteilhaften Ausführungsform weist die Vorrichtung wenigstens eine Abdichteinrichtung auf, welche die Bewegung der Behältnisse in deren Längsrichtung und/oder die Drehung der Behältnisse um deren Längsrichtung abdichtet. Genauer bewirkt diese Abdichteinrichtung, dass der Reinraum in diesem Bereich, in den die Bewegungen eingebracht werden, abgedichtet wird. Vorteilhaft ist daher die Greifeinrichtung zum Greifen der Behältnisse durch eine axiale und eine radiale Dichtung von dem Außenraum bzw. einer unsterilen Umgebung getrennt.

Um eine hygienische bzw. aseptische Abdichtung des Hub-Dreh-Mechanismus zu erhalten, wird hier vorgeschlagen, dass eine Abdichteinrichtung - etwa in Form eines Elastomerbalgs - die axiale Abdichtung übernimmt. Vorteilhaft wird die radiale Abdichtung durch ein radiales Dichtelement ausgeführt.

Es wird darauf hingewiesen, dass die hier beschriebene Abdichtung auch unabhängig von der obigen Erfindung anwendbar ist, also insbesondere ohne die oben beschriebenen gleichzeitigen Bewegungen. Die Anmelderin behält sich vor, hierauf ein eigenes Schutzbegehren zu richten.

Da der Elastomerbalg keinerlei Querkräfte bzw. Momente überträgt, ist dieser bevorzugt mit einer zusätzlichen Drehmomentabstützung versehen, welche die Querkräfte, die durch die Rotation des Greifsystems erzeugt werden, abfangen kann. Bevorzugt ist eine derartige Drehmomentabstützung in ihrer axialen Position verschiebbar gelagert und trägt besonders bevorzugt auch die radiale Dichtung, welche die rotierende Achse des Greifsystems zu dem Reinraum hin abdichtet.

Daneben wäre es auch denkbar, einen Balg, beispielsweise Faltenbalg, aus Teflon und/oder ähnlichen in der Aseptik bekannten Materialien zu fertigen, der durch seine Eigensteifigkeit auch gegebenenfalls ohne zusätzliche Drehmomentabstützung der rotativen Querkräfte auskommen könnte. Vorteilhaft könnte eine derartige rotative Dichtung angrenzend zu einer axialen Dichtung platziert sein. Eine Drehmomentabstützung zur Erhöhung der Lebensdauer ist jedoch ebenfalls denkbar.

Bei einer weiteren vorteilhaften Ausführungsform weist die Vorrichtung wenigstens ein Reflektorelement zum Reflektieren von Ladungsträgern und insbesondere Elektronen auf, welches derart angeordnet ist, dass die Behältnisse zwischen der ersten Außenbeaufschlagungseinrichtung und diesem Reflektorelement transportierbar sind. Um die aufgebrachte Dosis zu erhöhen, ist es denkbar, hinter dem Kunststoffbehältnis eine Art Reflektor zu platzieren, welcher beispielsweise aus Stahl oder Edelstahl, bevorzugt sogar aus einem Material mit höherer Dichte als Stahl besteht oder zumindest eine dünne Schicht aus diesem Material auf einem Trägermaterial aufweist.

Bei einer weiteren vorteilhaften Ausführungsform ist das oben erwähnte Greifsystem als Spannhülse ausgestaltet. Diese Spannhülse kann durch eine Mündung des Kunststoffbehältnisses in dieses eingeführt werden und hält daher das Behältnis von innen her.

Bei einer weiteren vorteilhaften Ausführungsform ist diese Spannhülse derart gestaltet, dass für eine federnde Wirkung zum Greifen und Festhalten des Behältnisses keine weiteren Bauteile notwendig sind und bevorzugt die federnde Wirkung allein in der Ausführung und Materialwahl der Hülse begründet sind. Auf diese Weise kann beispielsweise ein elastisches Material verwendet werden, welches in die Mündungen der Kunststoffbehältnisse eingeführt wird.

Bei einer weiteren vorteilhaften Ausführungsform wird eine Drehgeschwindigkeit in Abhängigkeit von einem Abstand des zu behandelnden Behältnisses zu der Strahlungseinrichtung variiert um bevorzugt eine möglichst homogene Dosisverteilung zu erhalten.

Bei einer weiteren vorteilhaften Ausführungsform weist die Vorrichtung eine zweite Außenbeschlagungseinrichtung auf, welche ebenfalls die Außenwandung der Behältnisse mit Strahlung beaufschlagt. Dabei kann diese zweite Außenbeaufschlagungseinrichtung gegenüber der ersten Außenbeaufschlagungseinrichtung entlang des Transportpfades der Kunststoffbehältnisse versetzt sein. Auch wäre es möglich, dass diese zweite Außenbeaufschlagungseinrichtung bezüglich des Transportpfades der Behältnisse auf der anderen Seite angeordnet ist wie die erste Außenbeaufschlagungseinrichtung. So könnten die Behältnisse zwischen diesen beiden Außenbeaufschlagungseinrichtungen geführt werden. Es wäre jedoch auch möglich, dass die zweite Außenbeaufschlagungseinrichtung sowohl in der Umfangsrichtung bzw. entlang des Transportpfades sowohl versetzt als auch auf der gegenüberliegenden Seite bezüglich des Transportpfades der Behältnisse (verglichen mit der ersten Außenbeaufschlagungseinrichtung) angeordnet ist.

Bei einer weiteren vorteilhaften Ausführungsform weist die oben erwähnte Kopplungseinrichtung zwei ineinander greifende Zahnräder auf. Diese ineinander greifenden Zahnräder dienen zur Übertragung bzw. Ausführung der Drehbewegung der Greifelemente, an denen die Kunststoffbehältnisse angeordnet sind und damit auch der Kunststoffbehältnisse selbst.

Bevorzugt ist dabei ein Zahnrad gegenüber dem anderen Zahnrad unter Beibehaltung eines Eingriffes zwischen den Zahnrädern verschiebbar. So ist es möglich, dass eines der beiden Zahnräder als Langrad ausgeführt ist. Vorteilhaft ist dabei dieses Langrad in der Längsrichtung der Behältnisse stationär angeordnet und ein zweites Rad, welches beispielsweise direkt die Kunststoffbehältnisse antreibt, kann diesem gegenüber verschiebbar angeordnet sein.

Bevorzugt ist eine Ausführungsform der Vorrichtung zum Sterilisieren von Behältnissen, bei der die zu sterilisierenden Behältnisse Vorformlinge, insbesondere Vorformlinge für (d.h. zum Herstellen von) Behältnisse für Getränke und/oder andere fluide Medien, sind. Dies ermöglicht durch die im Vergleich zu fertigen Behältnissen deutlich geringere Ausdehnung (bzw. Größe, Länge und/oder Durchmesser) der Vorformlinge eine geringere zu sterilisierende Oberfläche. Dadurch ist es auch möglich, die Beaufschlagungseinrichtungen kleiner zu dimensionieren. Bevorzugt lassen sich auch Strahlungsmenge, Strahlungsintensität, Beschleunigungsspannung und/oder andere Parameter verbessern, so dass der Energiebedarf der Vorrichtung und/oder deren Herstell- bzw. Anschaffungskosten reduziert werden können.

Bevorzugt ist eine Ausführungsform der Vorrichtung zum Sterilisieren von Behältnissen bei der der Transportpfad zumindest in einem Abschnitt, in welchem die erste Außenbeaufschlagungseinrichtung zum Sterilisieren zumindest eines Abschnitts einer Außenwandung von Behältnissen angeordnet ist, gekrümmt ist. Durch diese Ausführungsform ist es möglich, Transporteinrichtungen wie z.B. Transportsteme zu verwenden, bei welchen der Transportpfad zumindest abschnittsweise im Wesentlichen mäanderförmig ausgebildet ist. Diese Ausführungsform ermöglicht weiterhin eine besonders kompakte Bauweise.

Bevorzugt ist vorgesehen, dass in der Vorrichtung zum Sterilisieren von Behältnissen auf der der ersten Außenbeaufschlagungseinrichtung gegenüberliegenden Seite des Transportpfades eine weitere Außenbeaufschlagungseinrichtung angeordnet ist, wobei die Außenbeaufschlagungseinrichtung und die weitere Außenbeaufschlagungseinrichtung besonders bevorzugt entlang des Transportpfades versetzt angeordnet sind.

Diese Ausführungsform mit zwei sich bezüglich des Transportpfads gegenüberliegenden Außenbeaufschlagungseinrichtungen ist vorteilhaft, da so auch ohne eine Rotation der Behältnisse um deren eigene Längsachse die Beaufschlagung des Behältnisses mit Ladungsträgern von beiden Seiten möglich ist. Die Transporteinrichtung kann daher deutlich vereinfacht werden und z.B. auf einen Mechanismus zur vollständigen Rotation der Behältnisse und deren Längsachse verzichtet werden. Sollte trotz der gegenüberliegenden Außenbeaufschlagungseinrichtungen die Sterilisation in den Randbereichen dennoch nicht ausreichend sein, ist eine Schwenkbewegung der Behältnisse denkbar. Dabei soll unter einer Schwenkbewegung im Wesentlichen eine Rotation des Behältnisses um Bruchteile einer vollständigen Umdrehung verstanden werden. Die dafür notwendige Steuerung ist wesentlich einfacher als die für vollständige Rotationen und gewährleistet dennoch die ausreichende Sterilisierung aller äußeren Oberflächenbereiche des Behältnisses.

Um zu vermeiden, dass Ladungsträger direkt aufeinander zu beschleunigt werden, was u.a. zu besonders hoher Ladungsträgerdichte im Überlappungsbereich der emittierten Ladungsträgerwolken oder zu Beschädigungen der Außenbeaufschlagungseinrichtungen durch die ständige Beaufschlagung von Ladungsträgern, welche von der gegenüberliegenden Außenbeaufschlagungseinrichtung in deren Richtung emittiert werden, führen könnte, sind die Außenbeaufschlagungseinrichtungen bevorzugt um einen Winkel zwischen 2 und 20°, besonders bevorzugt zwischen 5 und 10°, eines Krümmungskreises des Transportpfads, versetzt entlang des Transportpfades angeordnet.

Bevorzugt sind somit die Maxima der Ladungsträgerwolken, welche von den Außenbeaufschlagungseinrichtungen in Richtung des Transportpfades abgegeben werden, mindestens 2 cm, bevorzugt mindestens 5 cm, besonders bevorzugt mindestens 10 cm und bevorzugt höchstens 200 cm, bevorzugt höchstens 100 cm, besonders bevorzugt höchstens 50 cm, entlang der Transportrichtung der Behältnisse beabstandet angeordnet. Dadurch gelangen die Behältnisse während des Transports zunächst in den Einflussbereich einer Außenbeaufschlagungseinrichtung und werden von dieser mit Ladungsträgern beaufschlagt. Zu einem späteren Zeitpunkt, wenn das Behältnis in einen stromabwärts befindlichen Einflussbereich der anderen Außeneaufschlagungseinrichtung gelangt, wird es von dieser mit Ladungsträgern beaufschlagt. Dabei können sich die Einflussbereiche der beiden Außenbeaufschlagungseinrichtungen überlappen.

Bevorzugt ist mindestens eine Außenbeaufschlagungseinrichtung im Wesentlichen außerhalb eines Reinraums angeordnet. Als im Wesentlichen außerhalb des Reinraums soll in diesem Zusammenhang verstanden werden, dass ein Großteil der Außenbeaufschlagungseinrichtung von außen und ohne Öffnung des Reinraums - und damit ohne mögliche Kontamination des Reinraums - zugänglich ist, das Ladungsträgeraustrittsfenster jedoch in einer Wandung des Reinraums angeordnet ist, so dass die Ladungsträger aus der Außenbeaufschlagungseinrichtung in den Reinraum und auf das zu sterilisierende Behältnis emittiert werden können.

Vorteilhaft ist die Vorrichtung zum Sterilisieren von Behältnissen in einem Bereich des Transportpfades, in welchem die erste Außenbeaufschlagungseinrichtung angeordnet ist, zumindest abschnittsweise von einer Strahlungsabschirmungsvorrichtung mit mindestens einer äußeren Strahlungsabschirmungseinrichtung und einer inneren Strahlungsabschirmungseinrichtung umgeben, durch welche eine von der ersten Außenbeaufschlagungseinrichtung abgegebene Strahlung zumindest teilweise absorbierbar ist.

Die Ausführungsform mit zwei Strahlungsabschirmungseinrichtungen erlaubt es, einen Transportkanal auszubilden, welcher sich zwischen den beiden Strahlungsabschirmungseinrichtungen erstreckt und in welchem die Behältnisse transportiert werden können. Als innere Strahlungsabschirmungseinrichtung wird diesbezüglich eine Strahlungsabschirmungseinrichtung verstanden, welche Ladungsträger in Richtung einer Innenseite der Vorrichtung abschirmt.

Da sich in diesem Bereich vorteilhafterweise keine Personen aufhalten, kann diese Strahlungsabschirmungseinrichtung evtl. weniger stark ausgebildet sein als eine äußere Strahlungsabschirmungseinrichtung. Diese schirmt bevorzugt auch die Umgebung, in der sich evtl. auch - zumindest temporär - Personen aufhalten können, von den Ladungsträgern und/oder der Strahlung ab. Bei der bevorzugten Ausführungsform der Anordnung der Außenbeaufschlagungseinrichtungen entlang eines Abschnitts eines Kreisumfangs ist die innere Strahlungsabschirmungseinrichtung bezüglich des Transportpfades und eines Kreismittelpunkts radial innenliegend und die äußere Strahlungsabschirmungseinrichtung bezüglich des Transportpfades und eines Kreismittelpunkts radial außenliegend.

Die äußere Strahlungsabschirmungseinrichtung ist demnach bevorzugt auf einer radial außenliegenden Seite eines gekrümmten Transportpfades angeordnet. Da die Außenbeaufschlagungseinrichtungen bevorzugt die stärksten Ladungsträgererzeugungseinrichtungen darstellen, ist in deren Einflussbereich eine besonders starke Abschirmung notwendig. Bevorzugt weist die in diesem Bereich angeordnete Strahlungsabschirmungseinrichtung daher stärkere Abschirmungseigenschaften auf als eine Strahlungsabschirmungseinrichtung, welche entlang eines anderen Bereiches des Transportpfades angeordnet ist.

Durch die Krümmung des Transportpfades im Bereich der Außenbeaufschlagungseinrichtungen ist es möglich, dass durch die äußere Strahlungsabschirmungseinrichtung Strahlung und/oder Ladungsträger - bevorzugt mindestens zweifach, besonders bevorzugt mehrfach - reflektiert, bevorzugt in Richtung der zu sterilisierenden Behältnisse reflektiert bzw. umgelenkt werden.

Bevorzugt ist eine Vorrichtung zum Sterilisieren von Behältnissen bei der die Strahlungsabschirmungsvorrichtung einen während des Transports von Behältnissen entlang des Transportpfades stationären Anteil und einen anderen, während des Transports von Behältnissen entlang des Transportpfades gegenüber dem ersten Anteil relativbeweglichen Anteil, aufweist.

Durch diese Ausführungsvariante ist es möglich, dass Greif- bzw. Halteelemente der Bewegung des relativbeweglichen Anteils der Strahlungsabschirmungsvorrichtung folgen und sich (zumindest mit einer Vektorkomponente) parallel zu der Transportrichtung der Behältnisse bewegen.

Vorteilhaft ist die Geschwindigkeit des relativbeweglichen Anteils der Strahlungsabschirmungsvorrichtung zumindest in einem Abschnitt des Transportpfades auf eine Geschwindigkeit, mit der die Behältnisse entlang des Transportpfades bewegt werden, anpassbar.

Bevorzugt durchdringen die Greif- bzw. Halteelemente den relativbeweglichen Anteil der Strahlungsabschirmungsvorrichtung zumindest abschnittsweise, so dass ein Anteil der Greif- bzw. Halteelemente im Inneren des Transportkanals angeordnet ist und dort die Behältnisse greifen bzw. halten kann. Ein anderer Anteil der Greif- bzw. Halteeinrichtung ist bevorzugt außerhalb des Transportkanals angeordnet. Dieser außerhalb liegende Anteil kann beispielsweise wartungsintensive Komponenten aufweisen, so dass deren Wartung möglich ist, ohne den sterilen Transportkanal öffnen zu müssen. Bevorzugt befinden sich Elemente eines Hub-, Dreh-, Schwenk- und/oder Transportmechanismus außerhalb des sterilen Transportkanals.

Insbesondere bei der Behandlung von Vorformlingen ist es notwendig, den Hubmechanismus auf die geringen Dimensionen der Vorformlinge im Vergleich zu fertigen Behältnissen anzupassen.

Bevorzugt werden die Behältnisse zumindest im Bereich der Außensterilisation durch eine im Wesentlichen einteilige Spannhülse gehalten bzw. transportiert.

Bevorzugt ist eine Vorrichtung zum Sterilisieren von Behältnissen bei der die Innenbeaufschlagungseinrichtung zum Sterilisieren zumindest eines Abschnitts einer Innenwandung von Behältnissen eine Beschleunigungseinrichtung mit einer geringeren Beschleunigungsspannung aufweist als eine Beschleunigungseinrichtung der ersten Außenbeaufschlagungseinrichtung.

Die Innenbeaufschlagungseinrichtung muss zumindest teilweise in das Innere des Behältnisses eingeführt werden. Daher bestehen besondere Anforderungen an deren Dimensionierung, die starke Abschirmungen, große Transformatoren, voluminöse Kühlelemente und andere große Bauteile nachteilig machen. Insbesondere bei der Innensterilisation von Vorformlingen besteht aufgrund deren geringen Ausdehnung kein Bedarf an besonders stark beschleunigten Ladungsträgern. Oft weisen Vorformlinge einen Innendurchmesser von < 5 cm, < 3 cm oder sogar < 2 cm auf, so dass sie Ladungsträger keine großen Distanzen zwischen einem im Behältnisinneren befindlichen Ladungsträgeraustrittsfenster und der Innenoberfläche des Behältnisses zurücklegen müssen. Daher sind für die Innenbeaufschlagungseinrichtung in diesem Fall geringe Beschleunigungsspannungen ausreichend. Eine Reduzierung der Beschleunigungsspannung auf das erforderliche Maß bietet Vorteile in der Energieeffizienz, den Betriebskosten und den Aufwendungen für eine Ausreichende Abschirmung der Umgebung gegenüber Ladungsträgern und/oder Strahlung.

Demgegenüber emittiert die Außenbeaufschlagungseinrichtung Ladungsträger über einen größeren Bereich, so dass dort stärkere Beschleunigungsspannungen und/oder größere Ladungsträgermengen benötigt werden. Dementsprechend ist in deren Nähe eine stärkere Abschirmung vorteilhaft.

Um die Außenbeaufschlagungseinrichtung dennoch möglichst platzsparend ausführen zu können und gleichzeitig dennoch den gesamten benötigten Oberflächenbereich des Behältnisses mit Ladungsträgern beaufschlagen zu können, weist bei einer bevorzugten Ausführungsform der Vorrichtung zum Sterilisieren von Behältnissen zumindest die erste Außenbeaufschlagungseinrichtung, bevorzugt auch eine weitere Außenbeaufschlagungseinrichtung jeweils ein im Wesentlichen rechteckiges oder ovales Ladungsträgeraustrittsfenster auf, wobei bevorzugt zumindest eine Längsachse des Ladungsträgeraustrittsfensters gegenüber einer Längsachse des mit Ladungsträgern zu beaufschlagenden Behältnisses geneigt ist. Bevorzugt ist die gesamte Außenbeaufschlagungseinrichtung gegenüber einer Längsachse des mit Ladungsträgern zu beaufschlagenden Behältnisses geneigt. Bevorzugt ist das Ladungsträgeraustrittsfenster im Wesentlichen rechteckig.

Als gegenüber der Längsachse des mit Ladungsträgern zu beaufschlagenden Behältnisses geneigt sollen Ladungsträgeraustrittsfenster oder auch Außenbeaufschlagungseinrichtungen verstanden werden, bei denen eine Hauptachse gegenüber der Längsachse des mit Ladungsträgern zu beaufschlagenden Behältnisses geneigt ist. Als Hauptachsen sollen diesbezüglich Achsen verstanden werden, die eine Vorzugsrichtung bzw. maximale Ausdehnung in eine Richtung des Austrittsfensters oder des Außenbeaufschlagungseinrichtungsgehäuses bzw. senkrecht dazu stehende Achsen darstellen. Beispiele dafür sind die große Halbachse und die kleine Halbachse eines ovalen.(elliptischen) Austrittsfensters oder Außenbeaufschlagungseinrichtungsgehäuses, die Mittelsenkrechten eines rechteckigen Austrittsfensters oder Außenbeaufschlagungseinrichtungsgehäuses oder andere.

Bevorzugt sind auch die Hauptachsen der Außenbeaufschlagungseinrichtungen gegenüber einander geneigt.

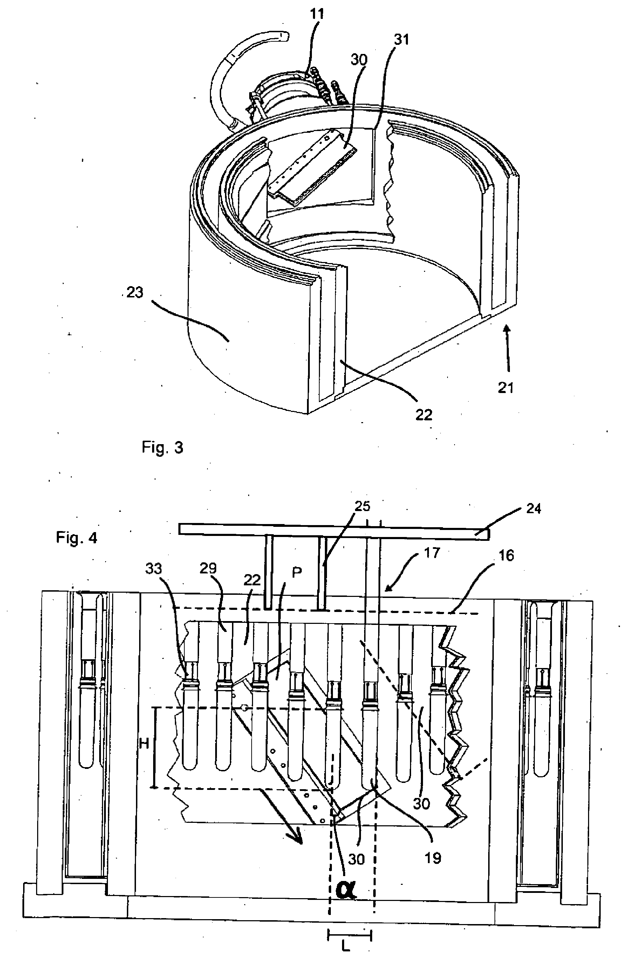

Durch die geneigte Anordnung der Außenbeaufschlagungseinrichtungen und/oder des Ladungsträgeraustrittsfensters kann der Abschnitt in dem ein Behältnis während dessen Transports entlang des Transportpfades sterilisiert wird, verlängert werden. Gleichzeitig kann aber auch gewährleistet werden, dass das Behältnis über dessen gesamte Höhe bzw. Länge jedoch mindestens über den gesamten zu sterilisierenden Bereich der äußeren Oberfläche den Ladungsträgern ausgesetzt ist. Wie detailliert bei der Beschreibung der Fig. 4 dargestellt ist, ergibt sich durch die Neigung des Ladungsträgeraustrittsfensters und/oder der Außenbeaufschlagungseinrichtung ein Bereich in Form eines Parallelogramms, in welchem sich die von dem Behältnis während dessen Transports überstrichene Fläche und die von der Außenbeaufschlagungseinrichtung emittierte Ladungsträgerwolke überschneiden.

Dieser Bereich ist so dimensioniert, dass das Behältnis diesen mit allen zu sterilisierenden Außenoberflächen durchläuft. Bevorzugt weist das diesen Bereich begrenzende Parallelogramm daher eine Höhe (über der Grundlinie) auf, die mindestens der Länge bzw. Höhe des Behältnisses entspricht. Durch die Neigung kann die Länge des Transportpfades, in welchem ein jeweiliger Anteil der äußeren Behältnisoberfläche sterilisiert wird, bevorzugt um ein Verhältnis vergrößert werden, welches (Streueffekte der Ladungsträger unberücksichtigt) ungefähr dem Sinus des Neigungswinkels zur Breite des Ladungsträgeraustrittsfensters entspricht.

Bevorzugt ist weiterhin eine Vorrichtung zum Sterilisieren von Behältnissen bei der die Vorrichtung eine Einführeinrichtung aufweist, mittels welcher die Innenbeaufschlagungseinrichtung zumindest abschnittsweise in das Innere eines Behältnisses einführbar ist, wobei das Behältnis und die zweite Ladungsträgeraustrittseinrichtung, bevorzugt in einer Längsrichtung des Behältnisses, gegenüber einander relativbeweglich sind.

In einer weiteren bevorzugten Ausführungsform der Vorrichtung zum Sterilisieren von Behältnissen kontaktiert ein Abschnitt einer gegenüber dem ersten Anteil der Strahlungsabschirmungsvorrichtung relativbeweglichen Trägerplatte zumindest zeitweise ein in einem Kanal befindliches Dichtungs- und/oder Sterilisationsmedium, um einen sich zumindest abschnittsweise entlang des Transportpfades erstreckenden Reinraum abzudichten. Die Trägerplatte ist bevorzug synchron zu einem gegenüber dem ersten Anteil der Strahlungsabschirmungsvorrichtung relativbeweglichen Anteil der Strahlungsabschirmungsvorrichtung bewegbar. Bevorzugt bildet die Trägerplatte eine Begrenzung des Reinraums.

Der Transportkanal ist demnach auf der Seite des relativbeweglichen Anteils der Strahlungsabschirmungsvorrichtung durch eine hydraulische Dichtung gegenüber der Umgebung abgeschlossen. Bevorzugt handelt es sich um einen Ring, welcher sich entsprechend der Bewegung der Behältnisse entlang des Transportpfades dreht und den Transportpfad, bzw. den Abschnitt des Transportpfades in welchem die Außensterilisation stattfindet abschirmt.

Dadurch kann gewährleistet werden, dass das Innere des Transportkanals gegenüber der Umgebung abgeschlossen ist und keine Fremdkörper, Verunreinigungen, Mikroorganismen, Sporen und andere in den Transportkanal eindringen können. Bevorzugt handelt es sich bei dem Medium um ein Medium, welches sowohl abdichtende als auch sterilisierende Eigenschaften aufweist. Weiter bevorzugt handelt es sich um ein flüssiges oder zähflüssiges (niederviskoses) Medium, so dass im Inneren des Transportkanals ein Überdruck ausgebaut werden kann, der das Einströmen von Umgebungsluft und darin mitgeführten Verunreinigungen in den sterilen Transportkanal verhindert. Es sind jedoch auch Gasse als Medium möglich, wobei diese bevorzugt derart in Strömung versetzt, bzw. gehalten werden, dass ein Eindringen von Fremdstoffen in den Reinraum verhindert wird. Insbesondere wird demnach eine Strömung aufrecht erhalten, welche aus Richtung des Reinraums in Richtung der Umgebung verläuft.

Bevorzugt ist unterhalb des Kanals, in welchem sich das Dichtungs- und/oder Sterilisationsmedium befindet ein weiterer Kanal angeordnet, in welchen Medium aus dem oberen Kanal eingebracht, z.B. eingesaugt, werden kann. Bei dem Dichtungs- und/oder Sterilisationsmedium handelt es sich bevorzugt um eine wässrige Lösung eines sterilisierenden Wirkstoffs (Sterilisationsmittels). Besonders bevorzugt wird als Dichtungsmittel ein Gasstrom verwendet, bevorzugt ein Luftstrom, insbesondere (gefilterte) Umgebungsluft, welcher durch seine Strömung das Eindringen von Verunreinigungen in das Innere des Reinraums verhindert.

Bevorzugt ist weiterhin eine Vorrichtung zum Sterilisieren von Behältnissen bei der der Abschnitt der gegenüber dem ersten Anteil der Strahlungsabschirmungsvorrichtung relativbeweglichen Trägerplatte zumindest zeitweise von dem in dem Kanal befindlichen Dichtungsund/oder Sterilisationsmedium trennbar ist, um, insbesondere zur Wartung und/oder Reinigung, einen Zugang zu dem sich entlang des Transportpfades erstreckenden Reinraum zu ermöglichen. Insbesondere ist vorgesehen, dass der gegenüber dem ersten Anteil relativbewegliche Anteil der Strahlungsabschirmungsvorrichtung, insbesondere ein Oberteil der Außensterilisationseinrichtung, nach oben abnehmbar ist. Dadurch ergibt sich die Möglichkeit, den zuvor gegenüber der Umgebung abgeschlossenen Transportkanal zu öffnen, um diesen zu reinigen. Beispielsweise kann so sehr einfach ein zusätzliches Desinfektions- bzw. Sterilisationsmittel in den Transportkanal eingeführt werden außerdem ist es möglich, evtl. in dem Transportkanal befindliche Behältnisse, welche sich von den Halteeinrichtungen gelöst haben, zu entfernen, um Beschädigung oder Verlust weiterer Behältnisse zu vermeiden.

Weiterhin ist eine Ausführungsform der Vorrichtung zum Sterilisieren von Behältnissen bei der die Vorrichtung eine Hubvorrichtung aufweist, mittels welcher die Behältnisse während deren Transport entlang des Transportpfades entlang deren Längsrichtung bewegbar sind. Bevorzugt befindet sich eine derartige Hubvorrichtung im Bereich einer Transporteinrichtung, welche die Behältnisse während deren Außensterilisation transportiert. Dadurch ist es möglich, die Behältnisse insbesondere während der Außensterilisation gegenüber der Außenbeaufschlagungseinrichtung auszurichten. Insbesondere ist es bei gegenüber der Behältnislängsachse geneigten Ladungsträgeraustrittsfenstern dadurch möglich, die Behältnisse jeweils so zu positionieren, dass der vorgesehene Bereich der äußeren Behältnisoberfläche mit Ladungsträgern beaufschlagt wird.

Bevorzugt weist dieser Hubmechanismus einen Motor und eine Gewindestangenführung auf, die dem geringen im Bereich der Außensterilisationseinrichtung zur Verfügung stehenden Platz angepasst sind. Die besagte Bewegung der Behältnisse in deren Längsrichtung wäre jedoch auch durch die Verwendung von Führungsschienen bzw. Steuerkurven realisierbar.

Bei den Ladungsträgern handelt es sich insbesondere um Elektronen, es wäre jedoch auch denkbar, dass andere Ladungsträger, wie Ionen, verwendet werden.

Das Ladungsträgeraustrittsfenster ist besonders bevorzugt aus einem Material hergestellt, welches aus einer Gruppe von Materialien ausgewählt ist, welche Titan, Quarzglas, Diamant, Kombinationen hieraus und dergleichen enthält.

Weiterhin ist eine Anlage zum Behandeln von Behältnissen Gegenstand der vorliegenden Erfindung, welche mindestens eine Vorrichtung der oben beschriebenen Art umfasst, wobei diese Vorrichtung bevorzugt stromabwärts bezüglich einer Heizeinrichtung zum Erwärmen von Kunststoffvorformlingen und stromaufwärts einer Abfülleinrichtung, bevorzugt stromaufwärts einer Umformungseinrichtung für Behältnisse angeordnet ist.

Durch eine derartige Anlage ist es möglich, eine Innen- und Außensterilisierung von Behältnissen insbesondere in den zur Produktion und Befüllung von Behältnissen verwendeten hohen Taktraten und Durchsatzzahlen durchzuführen.

Insbesondere wenn die beschriebene Vorrichtung stromabwärts bezüglich einer Heizeinrichtung und stromaufwärts bezüglich einer Umformungseinrichtung angeordnet ist, ist eine schnelle Sterilisation und ein schneller Transport durch die Vorrichtung notwendig. Dauert der Sterilisationsprozess bzw. der Transport durch die Sterilisationsvorrichtung zu lange, besteht die Gefahr des Abkühlens der Behältnisse, in diesem Fall der Vorformlinge. Bevorzugt ist die Vorrichtung daher geeignet, die Behältnisse innerhalb eines Zeitfensters von weniger als 20 Sekunden, bevorzugt weniger als 15 Sekunden, besonders bevorzugt etwa 11 Sekunden entlang des Transportpfades zu transportieren und dabei zu sterilisieren.

Bevorzugt weist die Anlage eine Transporteinrichtung auf, welche die Behältnisse entlang eines vorgegebenen Transportpfades insbesondere auch während deren Sterilisation bewegt. Vorteilhaft handelt es sich bei der Transporteinrichtung um einen drehbaren Träger, an dem besonders bevorzugt eine Vielzahl von Greifelementen angeordnet ist.

Vorzugsweise weist die Anlage eine Einrichtung zum Befüllen von Behältnissen auf und die erfindungsgemäße Vorrichtung ist stromaufwärts bezüglich dieser Einrichtung angeordnet.

Weiterhin ist bevorzugt, dass die Anlage mindestens ein Transportelement, bevorzugt einen Transportstern aufweist, welcher dazu geeignet ist, ein Behältnis von einer Vorrichtung zum Sterilisieren von Behältnissen zu übernehmen und an eine Vorrichtung zum Umformen der Behältnisse zu übergeben.

Bevorzugt weist eine solche Anlage jeweils einen sogenannten Schleusenstern zum Einführen bzw. zum Ausführen der Behältnisse in bzw. aus einer Vorrichtung der oben beschriebenen Art auf. Bevorzugt unterscheiden sich diese beiden Schleusensterne. Insbesondere aufgrund des innerhalb der Vorrichtung veränderten Teilungsabstands ist eine identische Ausführung der Schleusensterne nicht möglich.

Die vorliegende Erfindung ist weiteren auf ein Verfahren zum Sterilisieren von Kunststoffbehältnissen nach Anspruch 11 gerichtet, wobei die Behältnisse mittels einer Transporteinrichtung innerhalb eines Reinraumes transportiert werden und während dieses Transportes Außenoberflächen der Behältnisse durch Bestrahlung mit Ladungsträgern sterilisiert werden und wobei die Behältnisse wenigstens zeitweise in ihrer Längsrichtung bewegt werden.

Erfindungsgemäß werden die Behältnisse wenigstens zeitweise während ihrer Sterilisation um ihre Längsrichtung gedreht

Dabei ist es möglich, dass wenigstens zeitweise diese Rotation der Behältnisse und deren Bewegung in der Längsrichtung zeitgleich stattfinden. Es wäre jedoch auch möglich, dass zunächst eine Bewegung in der Längsrichtung der Behältnisse und anschließend eine Rotation stattfindet. Auch wäre es möglich, dass sich an diese Rotationsbewegung wiederum eine Bewegung in der Längsrichtung der Kunststoffbehältnisse anschließt.

So ist es möglich, dass die Strahlungseinrichtungen schräggestellt werden und auf diese Weise der Weg der Behältnisse an den Außenstrahlungseinrichtungen verlängert wird. Insbesondere finden dabei die Drehung und bevorzugt auch die Bewegung in der Längsrichtung während der Sterilisation statt.

Bei einem bevorzugten Verfahren werden die Bewegung der Behältnisse in deren Längsrichtung und/oder die Drehung der Behältnisse um deren Längsrichtung zur Aufrechterhaltung des Reinraumes abgedichtet.

Bevorzugt ist eine Variante des Verfahrens zum Sterilisieren von Behältnissen bei der Vorformlinge, insbesondere Vorformlinge für Getränkebehältnisse und/oder andere fluide Medien, sterilisiert werden. Dies hat den Vorteil, dass im Vergleich zu fertig ausgeformten Behältnissen wesentlich kleinere Oberflächen zu sterilisieren sind und der Prozess somit deutlich effizienter gestaltet werden kann. Außerdem kann die gesamte Vorrichtung kleiner dimensioniert sein, was in einer deutlichen Gewichtsreduzierung resultiert. Weiterhin wird dadurch eine höhere Prozessstabilität der Ladungsträgerbeaufschlagungseinrichtungen, also der Außenbeaufschlagungseinrichtung und der Innenbeaufschlagungseinrichtung, ermöglicht, da die eingesetzte Energie und die resultierende Fensterbelastung aufgrund der Abmessungen eines Vorformlings geringer sind.

Weiterhin bevorzugt ist eine Variante des Verfahrens zum Sterilisieren von Behältnissen bei dem das Behältnis entlang eines Transportpfads transportiert wird, welcher zumindest in einem Abschnitt, in welchem die Außenbeaufschlagungseinrichtung zum Sterilisieren zumindest eines Abschnitts einer Außenwandung von Behältnissen angeordnet ist, gekrümmt ist.

Außerdem ist eine Variante des Verfahrens zum Sterilisieren von Behältnissen bevorzugt auf bei welchem auf der der ersten Außenbeaufschlagungseinrichtung gegenüberliegenden Seite des Transportpfades, eine weitere Außenbeaufschlagungseinrichtung angeordnet ist, wobei die erste Außenbeaufschlagungseinrichtung und die Außenbeaufschlagungseinrichtung bevorzugt entlang des Transportpfades versetzt angeordnet sind. Bevorzugt sind die beiden Außenbeaufschlagungseinrichtungen um einen Winkel zwischen 2 und 20°, besonders bevorzugt zwischen 5 und 10°, eines Krümmungskreises des Transportpfads, gegenüber einander versetzt.

### Weitere Vorteile und Ausführungsformen ergeben sich aus den beigefügten Zeichnungen:

Darin zeigen:
Fig. 1 eine schematische Darstellung einer Aufsicht auf eine Vorrichtung zum Ste-rilisieren von Behältnissen;
Fig. 2 eine schematische Darstellung einer Aufsicht auf einen Bereich einer Vorrichtung zum Sterilisieren von Behältnissen in welchem die Außensterilisation der Behältnisse stattfindet;
Fig. 3 eine perspektivische Ansicht einer erfindungsgemäßen Vorrichtung zum Sterilisieren von Behältnissen;
Fig. 4 eine Seitenansicht zur Darstellung einer Vorrichtung zum Sterilisieren von Behältnissen;
Fig. 5 eine Darstellung eines Hub-Dreh-Mechanismus zum Bewegen der Kunststoffvorformlinge;
Fig. 6 eine Detaildarstellung des in Fig. 4 gezeigten Mechanismus;
Fig. 7 eine weitere Darstellung eines Drehmechanismus;
Fig. 8 eine weitere Detailansicht der erfindungsgemäßen Vorrichtung;
Fig. 9 eine weitere Draufsicht einer erfindungsgemäßen Vorrichtung;
Fig. 10 eine Schrägansicht der in Fig. 7 gezeigten Darstellung; und
Fig. 11 eine Gesamtdarstellung einer erfindungsgemäßen Vorrichtung.

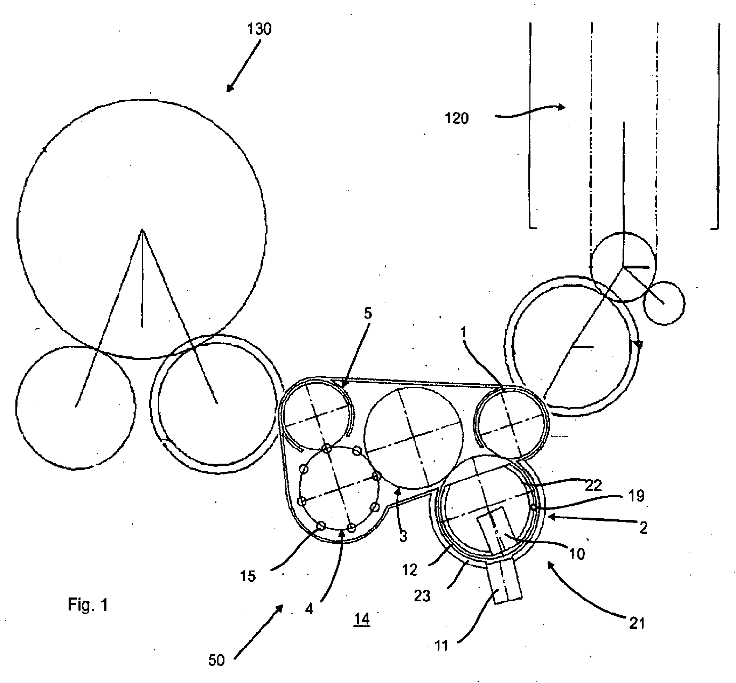

Figur 1 zeigt eine schematische Darstellung einer Aufsicht auf eine Vorrichtung 50 zum Sterilisieren von Behältnissen 19. Diese weist eine Transporteinrichtung 1 zum Aufnehmen von Behältnissen 19 auf, welche in der gezeigten Ausführungsform als Einlaufstern 1 ausgeführt ist. Stromabwärts bezüglich des Einlaufsterns 1 befindet sich eine Behandlungszone für die Behältnisse 19 in der die Außensterilisation stattfindet. Um die Behältnisse 19 mit Ladungsträgern beaufschlagen zu können, werden die Behältnisse an eine Transporteinrichtung 2 übergeben die die Behältnisse an den Außenbeaufschlagungseinrichtungen 10 und 11 vorbei transportiert. Während des Transports werden die Behältnisse 19 mit Ladungsträgern beaufschlagt, welche von den Außenbeaufschlagungseinrichtungen 10 und 11 emittiert werden. Um eine vollumfängliche Sterilisation zu ermöglichen, sind zwei Außenbeaufschlagungseinrichtungen 10 und 11 vorgesehen, wobei eine erste Außenbeaufschlagungseinrichtung 10 auf der radial innenliegenden Seite des Transportpfades angeordnet ist und eine zweite Außenbeaufschlagungseinrichtung 11 auf der radial außenliegenden Seite des Transportpfades. Denkbar wäre aber auch auf eine der beiden Außenbeaufschlagungseinrichtungen zu verzichten und stattdessen durch eine (z.B. vollständige) Rotation des Behältnisses eine vollständige Sterilisation des Behältnisses zu erreichen.

Zur Abschirmung der Umgebung 14 gegenüber der von den Außenbeaufschlagungseinrichtungen 10 und 11 emittierten Strahlung, insbesondere von der nach außen strahlenden Außenbeaufschlagungseinrichtung 10, ist dieser Bereich von einer starken Strahlungsabschirmungsvorrichtung 21 umgeben. Dabei setzt sich die Strahlungsabschirmungsvorrichtung aus mehreren Strahlungsabschirmungseinrichtungen 22 und 23 zusammen, welche den Transportkanal in verschiedene Richtungen abschirmen. In der gezeigten Aufsicht ist eine äußere Strahlungsabschirmungseinrichtung 23 und eine innere Strahlungsabschirmungseinrichtung 22 zu erkennen. Abschirmungen nach unten und oben sind nicht gezeigt.

Durch die Krümmung des Transportpfades und damit auch der Strahlungsabschirmungsvorrichtung 21 im Bereich der Außenbeaufschlagungseinrichtungen 10 und 11 ist es möglich, dass Strahlung zwischen der äußeren Strahlungsabschirmungseinrichtung 23 und der inneren Strahlungsabschirmungseinrichtung 22 reflektiert wird und so der Bereich, in dem die Sterilisation stattfindet größer ist als lediglich der Bereich unmittelbar vor den Außenbeaufschlagungseinrichtungen 10 und 11 bzw. deren Ladungsträgeraustrittsfenster.

Um eine möglichst lange Verweilzeit der Behältnisse in dem Bereich zu ermöglichen, in welchem die Sterilisation deren äußerer Oberfläche stattfindet, ist eine geringe Transportgeschwindigkeit und eine geringer Abstand zwischen den Behältnissen vorteilhaft. Durch den geringen Abstand zwischen zwei benachbarten Behältnissen entlang des Transportpfades ist auch bei geringer Transportgeschwindigkeit ein hoher Durchsatz zu erzielen. Weiterhin ist bei einem geringen Teilungsabstand vorteilhaft, dass wenige Ladungsträger (oder auch Strahlung) für den Sterilisationsprozess ungenutzt zwischen den Behältnissen hindurch passieren können.

Um jedoch einen größeren Teilungsabstand, also einen größeren Abstand zwischen zwei direkt aufeinander folgenden Behältnissen entlang des Transportpfades, wie er bei stromabwärts benötigten Einrichtungen benötigt wird, wieder herzustellen, ist stromabwärts direkt nach der Außensterilisationsvorrichtung eine Abstandsänderungseinrichtung 3, zur Veränderung eines Abstands zwischen zwei entlang des Transportpfades aufeinander folgenden Behältnissen angeordnet. Mittels dieser Abstandsänderungseinrichtung 3, in diesem Fall ein Teilungsverzugsstern, ist es möglich, den Abstand der Behältnisse so zu variieren, dass eine Übergabe an eine stromabwärts angeordnete Innsnsterilisationseinrichtung 15 möglich ist.

Die Innensterilisationseinrichtung weist eine Vielzahl von Innenbeaufschlagungseinrichtungen 15 auf, welche zumindest teilweise in die Behältnisse eingeführt werden können. Diese sind entlang bzw. um einer Transporteinrichtung 4 angeordnet, welche die Behältnisse während der Behandlung einer inneren Oberfläche der Behältnisse entlang des Transportpfades transportiert. Zur Innensterilisation weist jede der Innenbeaufschlagungseinrichtungen 15 einen sogenannten Strahlfinger auf, der so dimensioniert ist, dass er durch eine Öffnung des Behältnisses passt. Der übrige Teil jeder Innenbeaufschlagungseinrichtung 15 ist üblicherweise jedoch deutlich größer als der Strahlfinger und weist insbesondere einen größeren Durchmesser auf. Der Durchmesser ist üblicherweise auch größer als der jedes einzelnen zu sterilisierenden Behältnisses, so dass der Abstand zwischen zwei entlang des Transportpfades direkt aufeinander folgender Behältnisse im Bereich der Innensterilisationseinrichtung nicht mehr durch den Durchmesser der Behältnisse, sondern durch einen minimalen Abstand zwischen zwei benachbarten Innenbeaufschlagungseinrichtungen 15 vorge-geben ist. Insbesondere bei der Innensterilisation von Vorformlingen ist es daher notwendig, den Abstand zwischen zwei entlang des Transportpfades direkt aufeinander folgenden Behältnisse zu vergrößern und auf den Abstand zwischen zwei benachbarten Innenbeaufschlagungseinrichtungen 15 anzupassen.

Von der Innensterilisationseinrichtung werden die Behältnisse nach der Behandlung mit einer Innenbeaufschlagungseinrichtung 15 an eine stromabwärts angeordnete Transporteinrichtung 5 übergeben. Diese Transporteinrichtung 5 ist wie auch die Transporteinrichtung 1 als Transportstern 5 ausgeführt. Die Transportsteme 1 und 5 unterscheiden sich jedoch in Ihrem Aufbau. Mindestens der Teilungsabstand, mit welchem sie die Behältnisse transportieren, ist unterschiedlich. Der Transportstern 5 übernimmt die auf einer inneren Oberfläche sterilisierten Behältnisse von einer Transporteinrichtung 4, die die Behältnisse während der Innensterilisation transportiert und übergibt sie an eine weitere (nicht gezeigte) Transporteinrichtung oder eine Behältnisbehandlungseinrichtung. Beispielsweise könnte es sich bei einer stromabwärts des Transportsterns 5 angeordneten Behältnisbehandlungseinrichtung um eine Umformungseinrichtung oder um eine Abfülleinrichtung handeln. Die einzelnen Innenbeaufschlagungseinrichtungen 15 beaufschlagen vorteilhaft die gesamte Innenoberfläche der Behältnisse 19 mit Ladungsträgern.

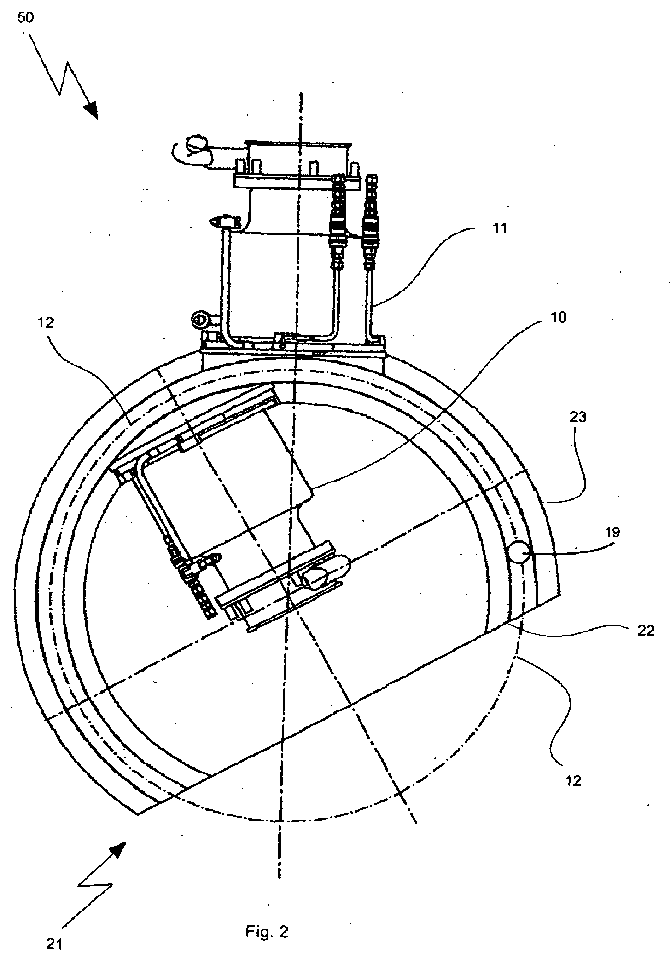

In Figur 2 ist eine schematische Darstellung einer Aufsicht auf einen Bereich einer Vorrichtung 50 zum Sterilisieren von Behältnissen 19 in welchem die Außensterilisation der Behältnisse 19 stattfindet gezeigt.

Insbesondere zeigt Fig. 2 einen Abschnitt des Transportpfades, entlang welchem die Außensterilisation der Behältnisse 19 stattfindet. Zur Außensterilisation werden die Behältnisse an den Außenbeaufschlagungseinrichtungen 10 und 11 vorbeigeführt. Diese Außenbeaufschlagungseinrichtungen 10 und 11 sind auf verschiedenen Seiten des Transportpfades angeordnet und sind somit dazu geeignet, ein Behältnis 19 von verschiedenen Seiten mit Ladungsträgern zu beaufschlagen. Dadurch kann der Transportmechanismus für die Behältnisse 19 vereinfacht werden, da eine vollständige Drehung der Behältnisse 19 zur vollumfänglichen Ladungsträgerbeaufschlagung nicht notwendig ist. Vorteilhaft beaufschlagen die Außenbeaufschlagungseinrichtungen 10 und 11 die gesamte äußere Umfangsfläche der Behältnisse 19 mit Ladungsträgern.

Die beiden Außenbeaufschlagungseinrichtungen 10 und 11 stehen sich nicht direkt gegenüber, sondern sind gegenüber einander entlang des Transportpfades der Behältnisse 19 versetzt angeordnet. Bei einem Transport der Behältnisse entlang des Transportpfades, welcher bei der in Fig. 2 gezeigten Aufsicht im Uhrzeigersinn (rechtsdrehend) verläuft, gelangen die Behältnisse somit zunächst in den Einflussbereich der radial innenliegenden und Ladungsträger radial nach außen beschleunigenden Außenbeaufschlagungseinrichtung 10.

Dort wird eine bezüglich der Transporteinrichtung 2 radial innenliegenden Außenoberfläche des Behältnisses mit Ladungsträgern beaufschlagt.

Erst etwas weiter stromabwärts gelangen die Behältnisse 19 in den Einflussbereich der radial außenliegenden und Ladungsträger radial nach innen beschleunigenden Außenbeaufschlagungseinrichtung 11. Die bei der Behandlung mit der anderen Außenbeaufschlagungseinrichtung 10 im Ladungsträgerschatten des Behältnisses liegende, bezüglich der Transporteinrichtung 2 radial außenliegende, Außenoberfläche des Behältnisses wird in diesem Bereich durch die Außenbeaufschlagungseinrichtung 11 mit Ladungsträgern beaufschlagt.

Die beiden Außenbeaufschlagungseinrichtungen 10 und 11 sind bezüglich des Zentrums bzw. der Rotationsachse der Transporteinrichtung 2 um einen vorgegebenen Winkel versetzt angeordnet. Durch diesen Versatz kann gewährleistet werden, dass die Ladungsträger beider Außenbeaufschlagungseinrichtungen 10 und 11 nicht direkt aufeinander zu beschleunigt werden, wodurch im Dauerbetrieb evtl. Beschädigungen der Außenbeaufschlagungseinrichtungen 10 und 11 hervorgerufen werden könnten. Außerdem können die Behältnisse 19 bei der Beaufschlagung mit den Ladungsträgern erwärmt werden, indem sie die kinetische Energie der beschleunigten Ladungsträger zumindest teilweise absorbieren und in thermische Energie umwandeln. Durch die versetzte Anordnung der Außenbeaufschlagungseinrichtungen 10 und 11 kann somit eine Überhitzung und damit eine mögliche Beschädigung der Behältnisse bei gleichzeitiger Ladungsträgerbeaufschlagung aus zwei Außenbeaufschlagungseinrichtungen 10 und 11 vermieden werden.

Darüber hinaus wird die Länge des Abschnitts des Transportpfades, in welchem die Behältnisse mit Ladungsträgern beaufschlagt werden, verlängert. Bei einer wolkenartigen Ausbreitung der Ladungsträger, bei welcher die Ladungsträger zusätzlich zu durch die Ladungsträgerbeschleunigungseinrichtung vorgegebenen Vorzugsrichtung auch einer Diffusion unterliegen, ist somit auch eine Überlappung der jeweils emittierten Ladungsträgerwolken möglich, so dass sich die Behältnisse 19 insgesamt länger in der gemeinsamen Ladungsträgerwolke beider Außenbeaufschlagungseinrichtungen 10 und 11 befinden.

Zur Abschirmung der Umgebung 14 gegenüber den emittierten Ladungsträgern und/oder bei der Ladungsträgererzeugung entstehender Strahlung ist der Bereich der Außensterilisation mit einer starken Strahlungsabschirmungsvorrichtung 21 umgeben. Diese besteht mindestens aus den Strahlungsabschirmungseinrichtungen 22 und 23, welche den Transportpfad auf verschiedenen Seiten umgeben. Bezüglich des Zentrums der Transporteinrichtung 2 (insbesondere einer Drehachse der Transporteinrichtung 2) ist eine Strahlungsabschirmungseinrichtung 23 radial außerhalb des Transportkanals angeordnet und eine Strahlungsabschirmungseinrichtung 22 radial innerhalb des Transportkanals angeordnet. Abschirmungen des Transportkanals senkrecht zur Zeichnungsebene sind nicht gezeigt.

Die Strahlungsabschirmungsvorrichtung 21 folgt im Bereich der Außenbeaufschlagungseinrichtungen 10 und 11 der Krümmung des Transportpfades und ermöglicht somit, dass Strahlung zwischen der äußeren Strahlungsabschirmungseinrichtung 23 und der inneren Strahlungsabschirmungseinrichtung 22 reflektiert wird. Dadurch kann der Bereich, in welchem die Behältnisse Ladungsträgern und/oder energiereicher (sterilisierender) Strahlung ausgesetzt sind, vergrößert werden.

Fig. 3 zeigt eine schematische Darstellung einer Schrägansicht auf einen Bereich einer Vorrichtung zum Sterilisieren von Behältnissen in welchem die Außensterilisation der Behältnisse 19 stattfindet mit zusätzlicher Darstellung eines Ladungsträgeraustrittsfensters 30.

Wie auch in Fig. 2 sind der von der Strahlungsabschirmungsvorrichtung 21 umgebene Transportkanal und eine Außenbeaufschlagungseinrichtung 11 gezeigt. Die bezüglich des Zentrums der (nicht gezeigten) Transporteinrichtung 2 radial innenliegende Außenbeaufschlagungseinrichtung 10 sowie der in diesem Bereich befindliche Teil der inneren Strahlungsabschirmungseinrichtung 22 sind nicht gezeigt, um die Anordnung der Außenbeaufschlagungseinrichtung 11 und deren Ladungsträgeraustrittsfenster 30 darstellen zu können.

Wie in Fig. 3 zu erkennen ist, ist die äußere Strahlungsabschirmungseinrichtung 23 im Bereich der Außenbeaufschlagungseinrichtung 11 unterbrochen, um den in der Außenbeaufschlagungseinrichtung 11 erzeugten Ladungsträgern einen Eintritt in den Transportkanal zu ermöglichen. Dazu schließt ein Gehäuse der Außenbeaufschlagungseinrichtung 11 den Transportkanal bündig ab, um Kontaminationen des als Reinraum ausgeführten Transportkanals zu verhindern. Realisert wird der bündige Anschluss der Außenbeaufschlagungseinrichtung 11 durch ein Befestigungselement 31, in diesem Fall einen Befestigungsflansch. Um einen ungehinderten Durchgang der Ladungsträger in den Reinraum bzw. Transportkanal zu ermöglichen, weist die Außenbeaufschlagungseinrichtung 11 ein Ladungsträgeraustrittsfenster 30 auf, durch welches Ladungsträger von einer Ladungsträgererzeugungseinrichtung hindurch in Richtung des Transportkanals beschleunigt werden können. Somit ist es möglich, dass die Ladungsträger durch das Ladungsträgeraustrittsfenster 30 in das Innere des Reinraums gelangen können und auf ein dort entlang des Transportpfades transportiertes Behältnis 19 treffen können.

Das Ladungsträgeraustrittsfenster 30 weist einen im Wesentlichen viereckigen Querschnitt auf. Auch wenn andere Geometrien des Ladungsträgeraustrittsfensters 30 wie Kreise, Ovale oder ein Quadrat als Spezialform eines Rechtecks ebenfalls möglich sind, ist eine Rechtecksform oder Parallelogrammform mit ungleichen Seitenlängen bevorzugt. Bei der gezeigten Rechtecksform sind die Hauptachsen gegenüber dem Transportpfad (bzw. dessen horizontalem Vektoranteil) geneigt ausgerichtet. Dadurch ist es möglich, dass sich ein an dem Ladungsträgeraustrittsfenster 30 vorbeigeführtes Behältnis 19 länger im Einflussbereich der Ladungsträgerwolke befindet. Dadurch ist bei vergleichbarer Sterilisationsleistung eine kompaktere Bauweise realisierbar.

Insbesondere dann, wenn durch einen Hubmechanismus das Behältnis 19 während des Transports entlang der Kreisbahn des Transportkanals angehoben werden kann und so dem Verlauf des Ladungsträgeraustrittsfensters 30 folgen kann ist eine Verlängerung des Behandlungsbereichs möglich.

Dies wird insbesondere in der in Fig. 4 gezeigten schematischen Darstellung einer Seitenansicht deutlich. Fig. 4 zeigt eine Seitenansicht eines Bereichs einer Vorrichtung 50 zum Sterilisieren von Behältnissen 19 in welchem die Außensterilisation der Behältnisse stattfindet mit zusätzlicher Darstellung der Ladungsträgeraustrittsfenster 30. Zur Verdeutlichung, dass die Ladungsträgeraustrittsfenster 30 mehrerer Außenbeaufschlagungseinrichtungen 10 und 11 gegenüber dem Transportpfad (bzw. dessen horizontalem Vektoranteil) und/oder der Behältnislängsachse um einen Winkel α geneigt angeordnet sein können, sind mehrere Ladungsträgeraustrittsfenster 30 gezeigt. Diese können auch gegenüber einander geneigt angeordnet sein. Dadurch ist es möglich, dass die im Bereich einer entlang des Transportpfades ersten Außenbeaufschlagungseinrichtung 10 angehobenen (bzw. in eine Richtung entlang der Behältnislängsachse bewegten) Behältnisse 19 im Bereich der entlang des Transportpfades folgenden Außenbeaufschlagungseinrichtung 11 wieder abgesenkt (bzw. in die entlang der Behältnislängsachse der ersten Bewegung entgegengesetzte Richtung bewegt) werden können.

Durch die gegenüber der Behältnislängsachse um den Winkel α geneigte Ausrichtung des Ladungsträgeraustrittsfensters 30 wird die Strecke, entlang welcher bei einem Transport des Behältnisses 19 dessen Außensterilisation stattfinden kann, verlängert. Bereits bei einer ausschließlichen Bewegung des Behältnisses 19 senkrecht zu dessen Längsachse, verlängert sich die Strecke des Transportpfades, entlang welcher ein Abschnitt der Außenwandung der Behältnisse 19 der Ladungsträgerbestrahlung ausgesetzt ist. Betrachtet man beispielsweise die seitliche Ummantelung der Basis eines Behältnisses 19, befindet sich diese entlang eines Abschnitts (Länge L des eingezeichneten Parallelogramms P) des Transportpfades in direktem Einfluss der senkrecht durch die Außenbeaufschlagungseinrichtung 11 auf das Behältnis abgegebener Ladungsträger. Diese Länge L ist (entsprechend den trigonometrischen Funktionen bzw. Winkelfunktion) gegenüber der Breite des Austrittsfensters um einen Faktor verlängert, welcher dem Kehrwert des Sinus des Neigungswinkels (also dem Kosekans des Neigungswinkels) entspricht.

Die Neigung des Ladungsträgeraustrittsfensters 30 ist dabei so gewählt, dass der Überlappungsbereich des Ladungsträgeraustrittsfensters 30 mit der während des Transports des Behältnisses 19 von dessen senkrechter Projektion auf dem Ladungsträgeraustrittsfenster 30 überstrichener Fläche, mindestens eine Höhe H aufweist, die der Länge des Behältnisses in dessen Längsrichtung entspricht. Im gezeigten Beispiel hat der Überlappungsbereich die Form eines Parallelogramms der Länge L und der Höhe H (über L). Je nach Form des Ladungsträgeraustrittsfensters 30, Ausrichtung des Behältnisses 19 bezüglich des Transportpfades und des Verlaufs des Transportpfades sind jedoch auch andere Geometrien des Überlappungsbereichs möglich.

Um eine besonders kompakte Ausführung der Vorrichtung zu ermöglichen, ist wie in Fig. 4 dargestellt, vorgesehen, dass das Behältnis 19 während dessen Transports entlang des Transportpfades zusätzlich auch (zumindest mit einer Vektorkomponente) entlang der Behältnislängsachse bewegbar ist. Wird das in Fig. 4 gezeigte Behältnis 19 während dessen Sterilisation nicht nur horizontal (bzw. parallel zur Länge L) bewegt, sondern zusätzlich auch in Höhenrichtung H bewegt, ist es möglich, das Behältnis noch länger im Einflussbereich der Ladungsträger zu halten.

Dazu weist die Transporteinrichtung 2 eine Hubeinrichtung 17 auf (nur schematisch dargestellt). Diese Hubeinrichtung 17 ist mit einer im Wesentlichen horizontalen Trägerplatte 24 verbunden, welche mit der Transporteinrichtung verbunden ist und deren horizontaler Bewegung, in diesem Fall der Rotationsbewegung der Transporteinrichtung 2, folgt. Die Hubeinrichtung 17 ist außerhalb des Reinraums angeordnet. Dadurch werden Wartungsarbeiten vereinfacht. Ein Halteelement 33, welches das Behältnis während dessen Transports entlang des Transportpfades trägt, befindet sich im Inneren des Reinraums und ist mit der Hubeinrichtung 17 verbunden. Die Verbindung zwischen Halteelement 33 und Hubeinrichtung 17 ist mittels eines Trägers 29 realisiert, welcher eine obere Strahlungsabschirmungseinrichtung 24 durchdringt. Die obere Strahlungsabschirmungseinrichtung 24 ist gegenüber der Strahlungsabschirmungseinrichtungen 22 und 23 relativbeweglich gelagert und bewegt sich parallel zu der Trägerplatte 24. Die Strahlungsabschirmungseinrichtung 24 weist daher Öffnungen auf, durch welche der Träger 29 geführt werden kann. Um die abschirmenden Eigenschaften der Strahlungsabschirmungseinrichtung 24 auch bei einer Bewegung des Trägers 29 bzw. des Behältnisses in dessen Längsrichtung aufrecht erhalten zu können, weist der Träger 29 zumindest abschnittsweise ein abschirmendes Material auf, durch welches die Öffnungen in der Strahlungsabschirmungseinrichtung 24 sowohl in Bezug auf Ladungsträger und Strahlungen, als auch in Bezug auf Kontaminationen des Reinraums abgedichtet werden können.

Um zu gewährleisten, dass die mit der Trägerplatte 24 verbundenen Hubeinrichtungen 17 und die Öffnungen in der Strahlungsabschirmungseinrichtung 16 derart aufeinander abgestimmt bewegen, dass eine Hubeinrichtung 17 über einer Öffnungen in der Strahlungsabschirmungseinrichtung 16 angeordnet ist, sind die Trägerplatte 24 und die Strahlungsabschirmungseinrichtung 16 über Trägerelemente 25 miteinander verbunden.

In Fig. 1 bezieht sich das Bezugszeichen 120 auf eine Erwärmungeinrichtung zum Erwärmen von Kunststoffvorformlingen und das Bezugszeichen 130 auf eine Umformungseinrichtung zum Umformen von Kunststoffvorformlingen zu Kunststoffbehältnissen. Man erkennt, dass hier die beiden Außensterilisationseinrichtungen 11 jeweils schräg gestellt sind bzw .deren Austrittsfenster schräg gestellt sind, wie dies bereits in Fig. 4 veranschaulicht wurde. Auch sind diese beiden Schrägstellungen gegensätzlich und bevorzugt achsensymmetrisch zu einer zwischen diesen beiden Austrittsfenstern verlaufenden Symmetrieachse. Auf diese Weise können, wenn man beispielsweise von einer Bewegung der Kunststoffvorformlinge im Uhrzeigersinn ausgeht, zunächst die Kunststoffvorformlinge an der rechten Außensterilisationseinrichtung vorbeigeführt werden und dabei gleichzeitig nach unten abgesenkt werden.

Im weiteren Verlauf können die Kunststoffvorformlinge wieder angehoben werden, wenn sie die linke Außensterilisationseinrichtung passieren. Gleichzeitig können die Kunststoffvorformlinge, wie oben erwähnt, durch die Antriebseinrichtungen 104 auch gedreht werden. Auf diese Weise wird eine sehr schnelle Sterilisation erreicht. An der entsprechenden Innenwandung 22 können, wie oben erwähnt, Reflektorelemente für die Elektronen angeordnet sein, um auch auf diese Weise die Bestrahlung der Kunststoffvorformlinge zu verbessern.

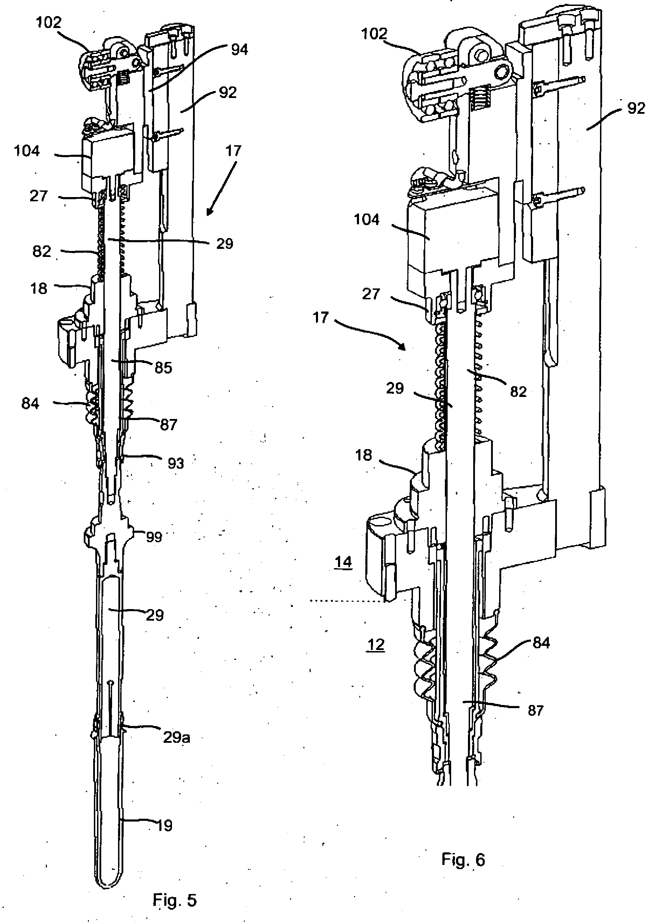

Figur 5 zeigt eine Darstellung zur Veranschaulichung des kombinierten Hub-Dreh-Mechanismus für die einzelnen Kunststoffvorformlinge 19. Diese sind dabei jeweils an einem Träger 29 und Halteeinrichtungen 29a angeordnet. Diese Halteeinrichtungen 29a greifen hier in die Mündungen der Kunststoffvorformlinge ein und sind bevorzugt gegen diese Innenwandung der Mündungen der Kunststoffvorformlinge 19 vorgespannt.

Das Bezugszeichen 84 kennzeichnet einen Faltenbalg, der zur Abdichtung einer Hubbewegung dient. Ein weiteres Trägerelement 85 erstreckt sich durch eine Reinraumgrenze hindurch. Das Bezugszeichen 82 kennzeichnet hier eine Druckfeder, die dazu dient, um die Kunststoffvorformlinge 19 anzuheben. Das Bezugszeichen 104 kennzeichnet eine Antriebseinrichtung, wie einen Antriebsmotor, der eine Drehbewegung der Kunststoffvorformlinge bewirkt. Das Bezugszeichen 102 kennzeichnet eine Kurvenrolle, die gegenüber einer (nicht gezeigten) Führungskurve abrollen kann, um so die Hubbewegungen durchzuführen. Das Bezugszeichen 92 bezeichnet einen Lagerblock, der zum Halten der Vorrichtung für die Durchführung der Hubbewegung dient. Entsprechend wird die Hubbewegung über ein Axiallager 94 ermöglicht. Am Ende des Faltenbalges 84 ist eine Lagerhülse 87 vorgesehen. Diese Hülse dient hier genauer als Einspannhülse und ist axial verschiebbar. Das Bezugszeichen 93 kennzeichnet eine rotative Dichtung. Durch die Anordnung können auf Wunsch die Drehbewegung und die Hubbewegung auch zeitgleich durchgeführt werden.

Das Bezugszeichen 18 kennzeichnet in ihrer Gesamtheit eine Axial- und Radiallagerung zur Lagerung der Drehwelle 85. Das Bezugszeichen 99 kennzeichnet ein Zwischenteil für eine Röntgenabschirmung. Zur Lagerung der Drehbewegung ist ein Drehlager 27 vorgesehen.

Figur 6 zeigt zur besseren Darstellung eine vergrößerte Ansicht der in Figur 5 gezeigten Anordnung.

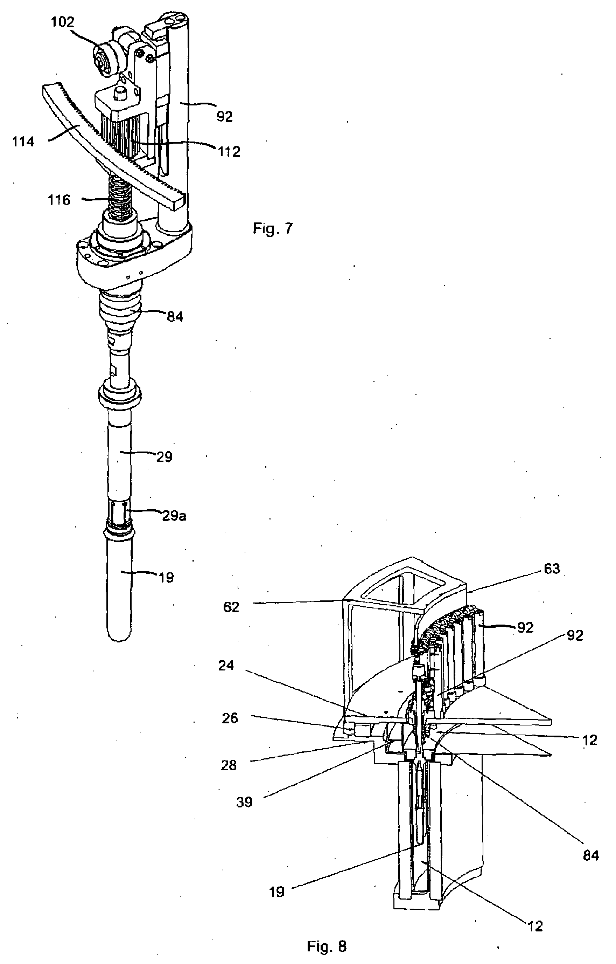

Figur 7 zeigt eine weitere Ausgestaltung eines möglichen Drehantriebes. Bei diesem Drehantrieb wird zwar wieder eine Kurvenrolle 102 zur Durchführung der Hubbewegung vorgesehen, hier wird jedoch die Drehbewegung über gegeneinander verschiebbare Verzahnungen ausgestaltet. Genauer gesagt ist hier ein erstes Zahnrad 112 vorgesehen, welches hier als Langrad ausgebildet ist. Weiterhin ist ein Hohlzahnrad 114 vorgesehen, welches in Eingriff mit dem Zahnrad 112 steht und auf diese Weise die Drehbewegung ermöglicht. Das Bezugszeichen 116 kennzeichnet wiederum eine Federungseinrichtung, welche zum Vorspannen der Hubbewegung dient und den Vorformling 19 nach oben zieht. Anstelle eines Hohlzahnrads kann auch ein Hohlzahnriemen vorgesehen sein, der hier ebenfalls eine Innenverzahnung aufweisen kann. Vorteilhaft ist ein derartiger (Hohl-)Zahnriemen flexibel ausgeführt. Daneben kann noch eine Antriebseinrichtung, wie ein Motor (nicht gezeigt) vorgesehen sein, welche dieses Hohlzahnrad bzw. einen entsprechenden Hohlzahnriemen antreibt.

Dadurch ist es möglich die Drehung des Preforms bzw. Behältnisses so zu beeinflussen, dass die Dosisverteilung gleichmäßig erfolgt. Dadurch behält man sich einerseits eine Beeinflussung der Drehung der Behältnisse vor und braucht andererseits nicht bei jeder Dreheinheit einen eigenen Servomotor vorzusehen.

Figur 8 zeigt eine weitere Darstellung der erfindungsgemäßen Vorrichtung. Hier ist auch die Hubkurve 63 dargestellt, die an einer Halterung 62 angeordnet ist. Das Bezugszeichen 12 kennzeichnet wiederum einen Reinraum, durch welchen die Kunststoffvorformlinge hindurchgeführt werden. Das Bezugszeichen 14 kennzeichnet die Umgebung dieses Reinraumes (welche unsteril ist).

Das Bezugszeichen 39 kennzeichnet einen Luftkanal und das Bezugszeichen 28 eine Trägerplatte, die zur Abstützung der Anordnung dient. Das Bezugszeichen 24 kennzeichnet eine weitere Trägerplatte für die Hubeinheit und das Bezugszeichen 26 einen Kugeldrehverbinder.

In der Regel werden die Kunststoffvorformlinge auf einem Stern an einem Emitter vorbeitransportiert und dabei entkeimt. Dabei ergibt sich ein wechselnder Abstand zwischen dem Kunststoffvorformling und dem in der Regel planaren Austrittsfenster der Strahlugseinrichtung. Um die Dosisunterschiede bei der Behandlung, hervorgerufen durch die unterschiedlichen Abstände, zu kompensieren, wäre es denkbar, die Rotation in Abhängigkeit von diesem Abstand des Kunststoffvorformlings zu dem Emitter durchzuführen. Dadurch wäre es auch möglich, die Dosisverteilung sehr homogen und gleichmäßig zu gestalten, obwohl der Abstand zu der Strahlungseinrichtung wechselt.

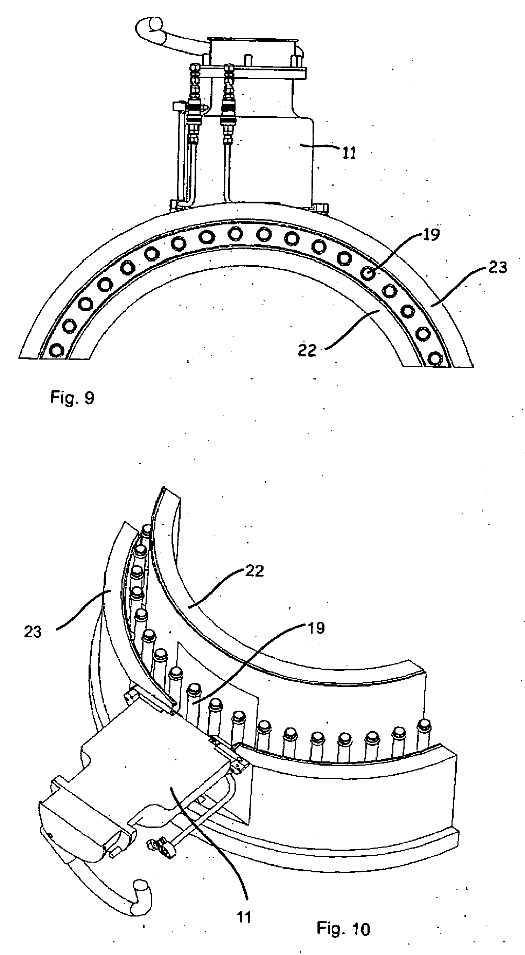

Die Figuren 9 und 10 zeigen zwei weitere vereinfachte Darstellungen einer erfindungsgemäßen Vorrichtung, wobei hier auch der Transportpfad der Vorformlinge 19 durch den Raum; der zwischen der radial inneren Strahlungsabschirmungseinrichtung und der radial äußeren Strahlungsabschirmungseinrichtung gebildet wird, veranschaulicht ist. Auch ist wieder die Außenbeaufschlagungseinrichtung 11 dargestellt.

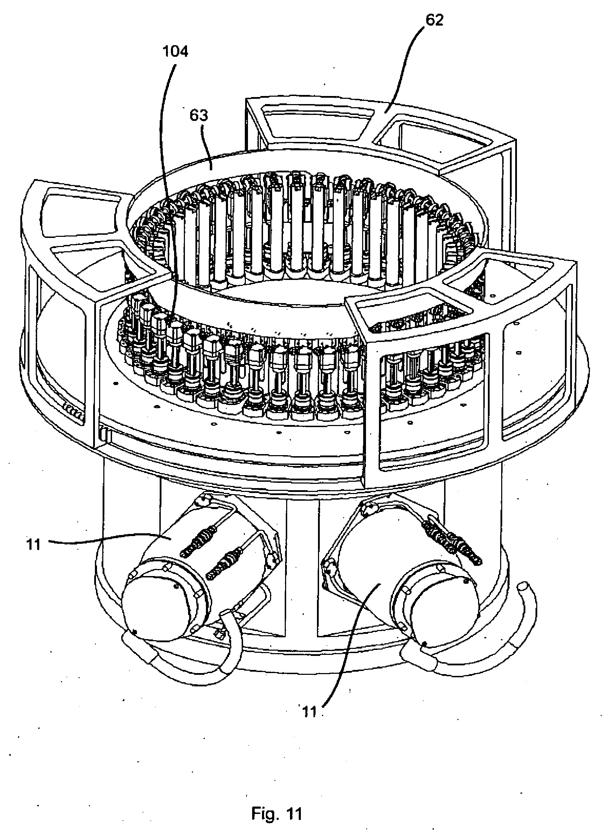

Figur 11 zeigt eine Gesamtdarstellung der erfindungsgemäßen Vorrichtung. Auch hier sind wieder die einzelnen Antriebe 104 zur Erzeugung der Drehbewegung erkennbar, sowie auch die Führungskurve 63, welche für die Hubbewegung verantwortlich ist. Für diese Ausführungsform sind zwei Außensterilisationseinrichtungen vorgesehen, die radial außerhalb des Transportpfades der Kunststoffvorformlinge angeordnet sind.
Die Außensterilisationseinrichtungen können dabei, wie oben erwähnt, Austrittsfenster aufweisen, durch welche die Ladungsträger bzw. Elektronen austreten können. Diese Austrittsfenster weisen dabei wenigstens abschnittsweise eine Folie, insbesondere eine Titanfolie auf. Diese Titanfolie weist dabei vorteilhaft eine Dicke zwischen 2 µm und 30 µm, bevorzugt zwischen 5 µm und 20 µm und besonders bevorzugt zwischen 6 µm und 14 µm auf. Vorteilhaft ist weiterhin eine Kühleinrichtung zum Kühlen dieser Austrittsfenster vorgesehen. Dabei sind Beaufschlagungseinrichtungen vorgesehen, welche die Austrittsfenster mit einem flüssigen und/oder gasförmigen Medium beaufschlagen. Daneben können auch Kühlgitter vorgesehen sein, an denen die Austrittsfenster anliegen. Diese Kühlgitter können von einem Kühlmedium durchflossen sein.

### Bezugszeichenliste

- 1: Einlaufstern
- 2: Transporteinrichtung
- 3: Abstandsänderungseinrichtung
- 4: Transporteinrichtung/Dreheinrichtung
- 5: Transporteinrichtung/Transportstern
- 10: Außenbeaufschlagungseinrichtung
- 11: Außenbeaufschlagungseinrichtung
- 12: Reinraum
- 14: Umgebung
- 15: Innensterilisationseinrichtung
- 16: Strahlungsabschirmungseinrichtung
- 17: Hubeinrichtung/Bewegungseinrichtung
- 18: Axial- und Radiallagerung
- 19: Behältnisse/Kunststoffvorformlinge
- 21: Strahlungsabschirmungseinrichtung
- 22: Strahlungsabschirmungseinrichtung
- 23: Strahlungsabschirmungseinrichtung
- 24: Trägerplatte
- 25: Trägerelement
- 27: Drehlager
- 29: Träger
- 29a: Halteeinrichtungen
- 30: Ladungsträgeraustrittsfenster
- 31: Befestigungselement
- 33: Haltelement
- 39: Luftkanal
- 50: Vorrichtung
- 60: Kopplungseinrichtung
- 62: Halterung
- 63: Führungskurve
- 82: Druckfeder
- 84: Faltenbalg
- 85: Drehwelle
- 87: Lagerhülse
- 92: Lagerblock
- 93: rotative Dichtung
- 94: Axiallager
- 99: Zwischenteil für Röntgenabschirmung
- 102: Kurvenrolle
- 104: Antriebseinrichtungen/Antriebe
- 112: Zahnrad
- 114: Holzzahnrad
- 116: Federungseinrichtung
- 120: Erwärmungseinrichtung
- 130: Umformungseinrichtung
- H: Höhe
- L: Länge/Längsrichtung
- U: Umgebung
- P: Parallelogramm
- α: Winkel

## Patentansprüche

1. Vorrichtung (50) zum Sterilisieren von Kunststoffbehältnissen (19) mit einer Transporteinrichtung (2), mittels welcher die Behältnisse (19) während deren Sterilisation entlang eines vorgegebenen Transportpfads transportierbar sind, mit einem Reinraum (12) innerhalb dessen die Behältnisse (19) während ihrer Sterilisierung transportierbar sind, wobei dieser Reinraum (12) mittels wenigstens einer Wandung gegenüber einer Umgebung abgegrenzt ist, mit einer ersten Aussenbeaufschlagungseinrichtung (11) zum Sterilisieren zumindest eines Abschnitts der Außenwandung der Behältnisse (19), wobei diese Aussenbeaufschlagungseinrichtung (11) eine Ladungsträgerquelle zum Erzeugen von Elektronen oder Ionen aufweist und mit einer ersten Hubeinrichtung (17), welche die Behältnisse (19) wenigstens zeitweise während ihrer Sterilisation in deren Längsrichtung (L) bewegt,
**dadurch gekennzeichnet, dass**
die Vorrichtung (1) eine Dreheinrichtung (4) aufweist, welche die Behältnisse wenigstens zeitweise während deren Sterilisation um deren Längsrichtung (L) dreht, wobei die Dreheinrichtung (4) und die Hubeinrichtung (17) derart ausgestaltet sind, dass eine Drehung der Behältnisse (19) während einer Bewegung der Behältnisse (19) in ihrer Längsrichtung (L) ermöglicht ist, wobei die Vorrichtung (50) eine mechanische Kopplungseinrichtung aufweist, welche die Bewegung der Behältnisse (19) in deren Längsrichtung (L) und die Drehbewegung der Behältnisse (19) um deren Längsrichtung miteinander koppelt.

2. Vorrichtung (50) nach Anspruch 1,
**dadurch gekennzeichnet, dass**
wenigstens ein Antriebsmittel der Dreheinrichtung (4) außerhalb des Reinraums (12) angeordnet ist.

3. Vorrichtung (50) nach wenigstens einem der vorangegangenen Ansprüche,
**dadurch gekennzeichnet, dass**
wenigstens ein Antriebsmittel der Hubeinrichtung (17) außerhalb des Reinraums (12) angeordnet ist.

4. Vorrichtung (50) nach wenigstens einem der Ansprüche 2 - 3,
**dadurch gekennzeichnet, dass**
die Vorrichtung wenigstens eine Abdichteinrichtung aufweist, welche die Bewegung der Behältnisse (19) in deren Längsrichtung (L) und/oder die Drehung der Behältnisse (19) um deren Längsrichtung abdichtet.

5. Vorrichtung (50) nach wenigstens einem der vorangegangenen Ansprüche,
**dadurch gekennzeichnet, dass**
die Vorrichtung wenigstens ein Reflektorelement zum Reflektieren von Elektronen aufweist, welches derart angeordnet ist, dass die Behältnisse (19) zwischen der ersten Aussenbeaufschlagungseinrichtung (11) und dem Reflektorelement transportierbar sind.

6. Vorrichtung (50) nach Anspruch 1
**dadurch gekennzeichnet, dass**
die mechanische Kopplungseinrichtung zwei ineinander greifende Zahnräder (112, 114) aufweist.

7. Vorrichtung (50) nach Anspruch 6,
**dadurch gekennzeichnet, dass**
ein Zahnrad (112) gegenüber dem anderen Zahnrad (114) unter Beibehaltung eines Eingriffs zwischen den Zahnrädern (112, 114) verschiebbar ist.

8. Vorrichtung (50) nach wenigstens einem der vorangegangenen Ansprüche,
**dadurch gekennzeichnet, dass**
die Aussenbeaufschlagungseinrichtung (11) ein Austrittsfester (30) aufweist, durch welches beschleunigte Ladungsträger austreten können, wobei dieses Austrittsfenster (30) wenigstens abschnittsweise ein folienartiges Element aufweist.

9. Vorrichtung nach Anspruch 1,
**dadurch gekennzeichnet, dass**
die Kopplungseinrichtung einen flexiblen Zahnriemen aufweist.

10. Vorrichtung nach Anspruch 1,
**dadurch gekennzeichnet, dass**
die Kopplungseinrichtung wenigstens ein Kopplungselement aufweist, welches in zwei zueinander entgegengesetzte Bewegungsrichtungen bewegt werden kann.

11. Verfahren zum Sterilisieren von Kunststoffbehältnissen, wobei die Behältnisse (19) mittels einer Transporteinrichtung (2) innerhalb eines Reinraums (12) transportiert werden und während dieses Transports Aussenoberflächen der Behältnisse (19) durch Bestrahlung mit Elektronen oder Ionen sterilisiert werden und wobei die Behältnisse wenigstens zeitweise in ihrer Längsrichtung (L) bewegt werden,
**dadurch gekennzeichnet, dass**
die Behältnisse (19) wenigstens zeitweise während ihrer Sterilisation um ihre Längsrichtung (L) mittels einer Dreheinrichtung (4) gedreht werden, und wobei die Dreheinrichtung (4) und die Hubeinrichtung (17) derart ausgestaltet sind, dass eine Drehung der Behältnisse (19) während einer Bewegung der Behältnisse (19) in ihrer Längsrichtung (L) ermöglicht ist, wobei mittels einer mechanischen Kopplungseinrichtung die Bewegung der Behältnisse (19) in deren Längsrichtung (L) und die Drehbewegung der Behältnisse (19) um deren Längsrichtung miteinander gekoppelt wird.

12. Verfahren nach Anspruch 11,
**dadurch gekennzeichnet, dass**
Bewegung der Behältnisse (19) in deren Längsrichtung (L) und/oder die Drehung der Behältnisse (19) um deren Längsrichtung zur Aufrechterhaltung des Reinraums (12) abgedichtet werden.

## Claims

1. Device (50) for the sterilization of plastics material containers (19) with a transport device (2) by means of which the containers (19) can be transported during their sterilization along a predefined transport path, with a clean room (12) within which the containers (19) can be transported during their sterilization, wherein this clean room (12) is delimited by means of at least one wall with respect to an environment, with a first external application device (11) for sterilizing at least a section of the outer wall of the containers (19), wherein this external application device (11) has a source of charge carriers for generation of electrons or ions, and with a first stroke device (17) which moves the containers (19) at least intermittently in their longitudinal direction (L) during their sterilization, **characterised in that** the device (1) has a rotary device (4) which rotates the containers about their longitudinal direction (L) at least intermittently during their sterilization, wherein the rotary device (14) and the stroke device (17) are configured in such a way that a rotation of the containers (19) is enabled during a movement of the containers (19) in their longitudinal direction (L), wherein the device (50) has a mechanical coupling device which couples together the movement of the containers (19) in their longitudinal direction (L) and the rotary movement of the containers (19) about their longitudinal direction.

2. Device (50) as claimed in claim 1, **characterised in that** at least one drive element of the rotary device (4) is disposed outside the clean room (12).

3. Device (50) as claimed in at least one of the preceding claims, **characterised in that** at least one drive element of the stroke device (17) is disposed outside the clean room (12).

4. Device (50) as claimed in at least one of claims 2 to 3, **characterised in that** the device (50) has at least one sealing device which seals the movement of the containers (19) in their longitudinal direction (L) and/or the rotation of the containers (19) about their longitudinal direction.

5. Device (50) as claimed in at least one of the preceding claims, **characterised in that** the device has at least one reflector element for reflecting electrons, which is arranged in such a way that the containers (19) can be transported between the first external application device (11) and the reflector element.

6. Device (50) as claimed in claim 1, **characterised in that** the mechanical coupling device has two interengaging gears (112, 114).

7. Device (50) as claimed in claim 6, **characterised in that** one gear (112) can be displaced with respect to the other gear (114) whilst retaining an engagement between the gears (112, 114).

8. Device (50) as claimed in at least one of the preceding claims, **characterised in that** the external application device (11) has an exit window (30) through which accelerated charge carriers can exit, wherein this exit window (30) has at least in sections a film-like element.

9. Device as claimed in claim 1, **characterised in that** the coupling device has a flexible toothed belt.

10. Device as claimed in claim 1, **characterised in that** the coupling device has at least one coupling element which can be moved in two opposing directions of movement.

11. Method for the sterilization of plastics material containers, wherein the containers (19) are transported by means of a transport device (2) within a clean room (12) and during this transport external surfaces of the containers (19) are sterilized by irradiation with electrons or ions and wherein the containers are moved at least intermittently in their longitudinal direction (L), **characterised in that** the containers (19) are rotated about their longitudinal direction (L) at least intermittently during their sterilization by means of a rotary device (4) and wherein the rotary device (4) and the stroke device (17) are designed in such a way that a rotation of the containers (19) is enabled during a movement of the containers (19) in their longitudinal direction (L) wherein by means of a mechanical coupling device the movement of the containers (19) in their longitudinal direction (L) and the rotary movement of the containers (19) about their longitudinal direction are coupled to one another.

12. Method (50) as claimed in claim 11, **characterised in that** movement of the containers (19) in their longitudinal direction (L) and/or the rotation of the containers (19) about their longitudinal direction is sealed in order to maintain the clean room (12).

## Revendications

1. Dispositif (50) de stérilisation de contenants en matière plastique (19) comprenant un dispositif de transport (2) au moyen duquel les contenants (19) peuvent être transportés pendant leur stérilisation le long d'un trajet de transport prédéfini, comprenant une salle blanche (12) à l'intérieur de laquelle les contenants (19) peuvent être transportés pendant leur stérilisation, cette salle blanche (12) étant séparée de l'environnement au moyen d'au moins une paroi, un premier dispositif d'application extérieure (11) permettant de stériliser au moins une section de la paroi extérieure des contenants (19), ce dispositif d'application extérieure (11) comprenant une source de porteurs de charge permettant de générer des électrons ou des ions et un premier dispositif de levage (17), lequel déplace les contenants (19) dans la direction longitudinale (L) de ces derniers au moins par intermittence pendant leur stérilisation,
**caractérisé en ce que**
le dispositif (1) comprend un dispositif de rotation (4), lequel fait tourner les contenants au moins temporairement autour de la direction longitudinale (L) de ces derniers pendant leur stérilisation, le dispositif de rotation (4) et le dispositif de levage (17) étant configurés de telle manière qu'une rotation des contenants (19) pendant un mouvement des contenants (19) dans leur direction longitudinale (L) est permise, le dispositif (50) comprenant un dispositif de couplage mécanique, lequel accouple l'un à l'autre le mouvement des contenants (19) dans la direction longitudinale (L) de ces derniers et le mouvement rotatif des contenants (19) autour de la direction longitudinale de ces derniers.

2. Dispositif (50) selon la revendication 1,
**caractérisé en ce qu'**
au moins un moyen d'entraînement du dispositif de rotation (4) est agencé à l'extérieur de la salle blanche (12).

3. Dispositif (50) selon au moins l'une des revendications précédentes,
**caractérisé en ce qu'**
au moins un moyen d'entraînement du dispositif de levage (17) est agencé à l'extérieur de la salle blanche (12).

4. Dispositif (50) selon au moins l'une des revendications 2 à 3,
**caractérisé en ce que**
le dispositif comprend au moins un dispositif d'étanchéité, lequel étanchéifie le mouvement des contenants (19) dans la direction longitudinale (L) de ces derniers et/ou la rotation des contenants (19) autour de la direction longitudinale de ces derniers.

5. Dispositif (50) selon au moins l'une des revendications précédentes,
**caractérisé en ce que**
le dispositif comprend au moins un élément réflecteur permettant le réfléchissement d'électrons, lequel est agencé de telle manière que les contenants (19) peuvent être transportés entre le premier dispositif d'application extérieure (11) et l'élément réflecteur.

6. Dispositif (50) selon la revendication 1,
**caractérisé en ce que**
le dispositif de couplage mécanique comprend deux roues dentées (112, 114) s'engrenant l'une dans l'autre.

7. Dispositif (50) selon la revendication 6,
**caractérisé en ce que**
une roue dentée (112) est mobile par rapport à l'autre roue dentée (114), l'engrènement entre les roues dentées (112, 114) subsistant.

8. Dispositif (50) selon au moins l'une des revendications précédentes,
**caractérisé en ce que**
le dispositif d'application extérieure (11) comprend une fenêtre de sortie (30) par laquelle les porteurs de charge mis en accélération peuvent sortir, cette fenêtre de sortie (30) comprenant au moins en sections un élément de type feuille.

9. Dispositif selon la revendication 1,
**caractérisé en ce que**
le dispositif d'accouplement comprend une courroie dentée flexible.

10. Dispositif selon la revendication 1,
**caractérisé en ce que**
le dispositif d'accouplement comprend au moins un élément d'accouplement, lequel peut être déplacé dans deux sens de mouvement opposés l'un à l'autre.

11. Procédé de stérilisation de contenants en matière plastique, les contenants (19) étant transportés au moyen d'un dispositif de transport (2) à l'intérieur d'une salle blanche (12) et, pendant ce transport, les surfaces extérieures des contenants (19) étant stérilisées en étant irradiées par des électrons ou des ions, et les contenants étant déplacés au moins temporairement dans leur direction longitudinale (L),
**caractérisé en ce que**
les contenants (19) sont amenés en rotation au moins temporairement autour de leur direction longitudinale (L) au moyen d'un dispositif de rotation (4) pendant leur stérilisation, et le dispositif de rotation (4) et le dispositif de levage (17) étant conçus de telle manière qu'une rotation des contenants (19) pendant un mouvement des contenants (19) dans leur direction longitudinale (L) de ces derniers est permise, le mouvement des contenants (19) dans la direction longitudinale (L) de ces derniers et le mouvement de rotation des contenants (19) autour de la direction longitudinale de ces derniers étant accouplés l'un à l'autre au moyen d'un premier dispositif d'accouplement mécanique.

12. Procédé selon la revendication 11,
**caractérisé en ce que**
le mouvement des contenants (19) dans la direction longitudinale (L) de ces derniers et/ou la rotation des contenants (19) autour de la direction longitudinale de ces derniers sont étanchéifiés pour la préservation de la salle blanche (12).
